# FASCICULE DE BREVET EUROPEEN

(11) **EP 3 237 384 B1**
(45) Date de publication et mention de la délivrance du brevet: **30.01.2019**
(21) Numéro de dépôt: 15828354.9
(22) Date de dépôt: 22.12.2015
(51) Int. Cl.: C07D 213/75, A61K 31/145, A61K 31/17, A61K 31/341, A61K 31/381, A61K 31/4409, A61K 31/443, C07D 407/12, C07D 409/06, C07C 381/10, C07D 213/38, C07D 307/52, A61P 17/06, A61P 19/02, A61P 17/10, A61P 37/06, A61P 35/00

(54) **NOUVEAUX COMPOSÉS ANTAGONISTES DES RÉCEPTEURS CXCR1 ET CXCR2 AUX CHIMIOKINES, ET LEUR UTILISATION DANS LE TRAITEMENT DE PATHOLOGIES MÉDIÉES PAR DES CHIMIOKINES**
NEUARTIGE CHEMOKIN-CXCR1- UND -CXCR2-REZEPTORANTAGONISTENVERBINDUNGEN UND VERWENDUNG DAVON BEI DER BEHANDLUNG VON CHEMOKINVERMITTELTEN PATHOLOGIEN
NOVEL CHEMOKINE CXCR1 AND CXCR2 RECEPTOR ANTAGONIST COMPOUNDS, AND USE THEREOF IN THE TREATMENT OF CHEMOKINE-MEDIATED PATHOLOGIES

(30) Priorité: 23.12.2014 FR 1463209
(43) Date de publication de la demande: 01.11.2017
(73) Titulaire: Galderma Research & Development, 06410 Biot (FR)
(72) Inventeur: MUSICKI, Branislav, 06000 Nice (FR); BHURRUTH-ALCOR, Yushma, 06600 Antibes (FR)
(74) Mandataire: Cabinet Becker et Associés
(86) Numéro de dépôt international: PCT/FR2015/053703
(87) Numéro de publication internationale: WO 2016/102877

(56) Documents cités:
- WO-A1-00/35442
- WO-A1-02/057230
- WO-A1-02/083624
- WO-A1-2008/148790
- WO-A1-2010/015613

## Description

### Domaine de l'invention :

La présente invention porte sur de nouveaux composés antagonistes des récepteurs CXCR1 et CXCR2 aux chimiokines, sur les compositions pharmaceutiques contenant ces composés ainsi que sur l'utilisation de ces composés et de ces compositions pour le traitement de pathologies médiées par des chimiokines telles que définies dans la présente demande, plus particulièrement dans le domaine de la dermatologie.

### Etat de la technique antérieur à l'invention :

Les chimiokines ou cytokines sont de petites protéines solubles. Leur rôle le plus connu est l'attraction et le contrôle de l'état d'activation des cellules du système immunitaire. Toutes les chimiokines exercent leurs fonctions en se fixant sur des récepteurs couplés aux protéines G. Certaines chimiokines sont considérées comme pro-inflammatoires. La sécrétion de ces chimiokines peut être induite lors de la réponse immune afin de favoriser l'arrivée de cellules du système immunitaire au niveau d'un site infectieux.
Il existe deux types de chimiokines : les chimiokines pro-inflammatoires et les chimiokines constitutives.
Les chimiokines pro-inflammatoires (ou « inductibles ») sont produites au niveau de sites d'inflammation par des cellules de tissus ou des leucocytes infiltrés, après contact avec un agent pathogène.
Les chimiokines constitutives (ou « homéostatiques ») sont produites dans les organes lymphoïdes et dans certains organes non-lymphoïdes tels que la peau et les muqueuses. Elles régulent le trafic lymphocytaire et la localisation des lymphocytes au sein de ces organes pendant la lymphopoïèse mais également pour maintenir l'immunosurveillance.
La nomenclature de ces récepteurs à chimiokines est basée sur le groupe de chimiokines auquel son ligand appartient. Ainsi, les récepteurs correspondant aux chimiokines du groupe CXC sont, par exemple, appelés CXCR1, CXCR2, CXCR3, CXCR4, etc, et les récepteurs correspondant aux chimiokines du groupe CC sont, par exemple, appelés CCR1, CCR2, CCR3, etc. Ces récepteurs présentent tous une structure tertiaire semblable, et ils sont couplés à une protéine G : ils font donc partie de la superfamille des GPCR (G Protein Coupled Receptor).
L'interleukine-8 ou IL-8 (également nommé CXCL-8) est un membre de la famille des chimiokines CXC, qui joue un rôle primordial dans le recrutement des neutrophiles vers le site d'inflammation. Deux récepteurs, CXCR1 et CXCR2 sont connus pour être spécifiquement activés par IL-8. Alors que CXCR2 se lie avec une forte affinité à IL-8 et aux chimiokines apparentées comme CXCL6, CXCL5, CXCL3, CXCL2 et CXCL1, CXCR1 se lie uniquement à IL-8. Des niveaux élevés d'IL-8 et de chimiokines apparentées (CXCL5, CXCL2 & CXCL1) ont été décrits dans les lésions d'acné inflammatoire (J Invest Dermatol. 2006;126:1071-9; Am J Pathol. 2005;166(6):1691-9; Diagn Pathol. 2007 Jan 30;2:4).
Des premiers indices démontrent l'expression de CXCR2 dans l'acné inflammatoire (Trivedi et al. J Invest Dermatol. 2006 126(5):1071-9). Ainsi, des antagonistes doubles de CXCR1 et CXCR2 pourrait permettre de diminuer rapidement les effets délétères de la réponse inflammatoire par IL-8.

Il est aujourd'hui connu que de nombreuses pathologies d'ordre inflammatoire sont médiées par des chimiokines.

Dans le domaine de la dermatologie, il existe également un besoin non satisfait à ce jour de traiter la composante inflammatoire de pathologies d'intérêt comme par exemple l'acné, la rosacée ou encore les dermatoses neutrophiliques, notamment le psoriasis.

Or, la demanderesse a découvert de nouveaux composés présentant une activité antagoniste vis-à-vis des récepteurs de type CXCR1 et CXCR2 et se caractérisant par la présence dans leur structure d'un groupement fonctionnel, de type sulfoximine, répondant à la sous structure (la) suivante :

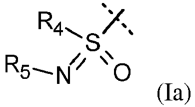

Comparativement à leurs analogues structuraux les plus proches, pris dans l'art antérieur, par exemple dans le document WO02/083624, et présentant un groupement fonctionnel de type sulfone à la place du groupement fonctionnel de type sulfoximine, ces composés présentent une meilleure activité antagoniste vis-à-vis des récepteurs de type CXCR1 et CXCR2.
Ils présentent également de façon inattendue une meilleure inhibition de la migration des neutrophiles sur le site inflammatoire comparativement à leurs analogues structuraux les plus proches, pris par exemple dans le document WO02/083624, ce qui leur confère un intérêt supplémentaire par rapport aux composés déjà connus dans le traitement de pathologies médiées par des chimiokines, et plus particulièrement des pathologies d'ordre dermatologique.

Par ailleurs, WO 2008/148790 divulgue des composés dérivés de cyclobutènedione ayant des propriétés anti-inflammatoires. WO 02/057230 divulgue des composés de type sulfonamides squaramides dans le traitement de maladies médiées par les chimiokines, telles que IL-8. WO 00/35442 divulgue des dérivés d'hydroxy diphénylurée substitués par un sulfonamide an tant qu'antagonistes des récepteurs IL-8. WO 2010/015613 divulgue des composés dérivés de cyclobutènedione et leur utilisation dans le traitement des maladies inflammatoires et des allergies.

### Résumé de l'invention :

La présente invention est telle que définie par l'objet des revendications 1 à 8.

Un premier objet selon l'invention concerne de nouveaux composés antagonistes des récepteurs CXCR1 et CXCR2 aux chimiokines, ainsi que leurs sels et énantiomères, répondant à la formule générale (I) suivante: 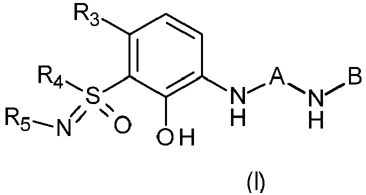 dans laquelle les groupes A et B et les substituants R3, R4, R5 sont tels que définis ci-après dans la description détaillée de l'invention.

Un second objet selon l'invention concerne une composition pharmaceutique comprenant une quantité efficace d'un composé répondant à la formule générale (I) en association avec un solvant ou un support pharmaceutiquement acceptable.

Un troisième objet selon l'invention concerne un composé ou une composition pharmaceutique tel que décrit ci-dessus pour son utilisation en tant que médicament.

Un quatrième objet selon l'invention concerne un composé ou une composition pharmaceutique tel que décrit ci-dessus pour son utilisation dans le traitement de maladies médiées par les chimiokines telles que définies dans la présente demande.

Un cinquième objet selon l'invention concerne un composé ou une composition pharmaceutique tel que décrit ci-dessus pour son utilisation dans le traitement de maladies médiées par les chimiokines sélectionnées dans le groupe comprenant les dermatoses neutrophiliques, et notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

La présente demande divulgue aussi une méthode pour traiter des maladies médiées par les chimiokines comprenant l'administration d'une quantité efficace d'un composé ou d'une composition pharmaceutique tels que définis dans la présente demande chez un patient souffrant d'une maladie médiée par les chimiokines.

La présente demande divulgue également l'utilisation d'un composé ou d'une composition pharmaceutique tels que définis dans la présente demande pour la préparation d'un médicament pour traiter des maladies médiées par les chimiokines.

### Description détaillée de l'invention :

La figure 1 représente le schéma général de synthèse permettant de préparer les composés de formule générale (I) dans laquelle R5 représente un hydrogène et B représente (B1).

La figure 2 représente le schéma général de synthèse permettant de préparer les composés de formule générale (I) dans laquelle R5 est différent de l'hydrogène et B représente (B1).

A moins qu'il en soit indiqué autrement, les définitions suivantes s'appliquent à l'ensemble de la description et des revendications.
Ces définitions s'appliquent indépendamment de savoir si un terme est utilisé seul ou en combinaison avec d'autres termes. Ainsi, par exemple, la définition du terme « aryle » s'applique aussi bien à « aryle » en tant que tel qu'à la partie « aryle » du terme « aryloxy » ou encore « arylalkyle ».

**"Alkyle"** désigne une chaîne hydrocarbonée saturée linéaire ou ramifiée dont le nombre d'atomes de carbone est précisé.
Lorsque le nombre d'atomes de carbone n'est pas précisé, cela signifie que la chaine alkyle contient de 1 à 20 atomes de carbone.
Les radicaux alkyles préférés contiennent de 1 à 12 atomes de carbone, et ceux encore plus préférés contiennent de 1 à 6 atomes de carbone dans la chaîne.

«**Alkoxy**" désigne un oxygène substitué par un radical alkyle tel que défini précédemment. Des exemples de radicaux alkoxy comprennent les radicaux méthoxy, éthoxy, n-propoxy, isopropoxy et n-butoxy.

**«Aryle»** désigne un système cyclique aromatique monocyclique ou polycyclique (2 à 3 cycles) comprenant de 6 à 14 atomes de carbone, et de préférence de 6 à 10 atomes de carbone.
A titre d'exemples de radical aryle, on peut citer le radical phényle, naphtyle, indényle, tétrahydronaphtyle, indanyle, anthracényle et fluorényle.
Les radicaux aryles préférés sont le radical phényle et le radical naphtyle. Le radical aryle encore plus préféré est le radical phényle.

**« Hétéroaryle »** désigne un système aromatique monocyclique ou polycyclique (2 à 3 cycles) comprenant de 5 à 14 atomes cycliques, de préférence de 5 à 10 atomes cycliques, dans lequel un ou plusieurs des atomes cycliques représente(nt) un ou plusieurs (de 1 à 5) hétéroatome(s) choisi(s) dans le groupe comprenant l'azote, l'oxygène et le soufre.
Les hétéroaryles préférés contiennent 5 ou 6 atomes cycliques et 1 à 3 hétéroatomes.

Le préfixe aza, oxa ou thia avant le nom de la racine hétéroaryle signifie qu'au moins un azote, un oxygène ou un soufre est respectivement présent dans le cycle.
Un atome d'azote d'un hétéroaryle peut être éventuellement oxydé en N-oxyde. A titre d'exemples d'hétéroaryles appropriés, on peut citer les hétéroaryles suivants :
pyridyle, pyrazinyle, furanyle, thiényle, pyrimidinyle, isoxazolyle, isothiazolyle, oxazolyle, thiazolyle, pyrazolyle, furazanyle, pyrrolyle, pyrazolyle, triazolyle, le 1,2,4-thiadiazolyle, pyrazinyle, pyridazinyle, quinoxalinyle, phtalazinyle, imidazo [1,2-a] pyridinyle, imidazo [2,1-b] thiazolyle, benzofurazanyl, indolyle, azaindolyle, benzimidazolyle, benzothiényle, quinolinyle, imidazolyle, thienopyridyle, quinazolinyle, thienopyrimidyle, pyrrolopyridyle, imidazopyridyle, isoquinolinyle, benzoazaindolyle, 1,2,4 triazinyle et benzothiazolyle.
Les radicaux hétéroaryles préférés sont choisis dans la liste suivante : pyridyle, pyrazinyle, furanyle, thiényle, pyrimidinyle, isoxazolyle, isothiazolyle, oxazolyle, thiazolyle, pyrazolyle, furazanyle, pyrrolyle, pyrazolyle, triazolyle.
Les radicaux hétéroaryles encore plus préférés sont le radical pyridyle et le radical furanyle.

«**Arylalkyle »** désigne un radical dont les parties aryle et alkyle sont telles que définies ci-dessus.
A titre d'exemples d'arylalkyle, on peut citer les radicaux benzyle, phénéthyle et naphthalènylméthyle.
La liaison à la structure à laquelle il est rattaché se fait par le radical alkyle.

**«Hétéroarylalkyle »** désigne un radical dont les parties hétéroaryle et alkyle sont telles que définies ci-dessus.
A titre d'exemples d'hétéroarylalkyle, on peut citer les radicaux pyridylméthyle, pyridyléthyle, imidazolylméthyle, imidazolyléthyle, pyrazolylméthyle et pyrazolyléthyle.
La liaison à la structure à laquelle il est rattaché se fait par le radical alkyle.

**«Cycloalkyle »** désigne un système cyclique hydrocarboné non aromatique, ayant de 3 à 10 atomes de carbone, de préférence de 5 à 10 atomes de carbone, et de un à trois cycles.
Les radicaux cycloalkyles préférés contiennent de 5 à 7 atomes cycliques.
A titre d'exemples de radicaux cycloalkyle, on peut citer les radicaux cyclopropyle, cyclopentyle, cyclohexyle, cycloheptyle, norbornyle et adamantyle.

**«Cycloalkylalkyle »** désigne un radical dont les parties cycloalkyle et alkyle sont telles que définies ci-dessus.
A titre d'exemples de cycloalkylalkyle, on peut citer les radicaux cyclopropylméthyle, cyclopropyléthyle, cyclobutylméthyle, cyclobutyléthyle, cyclopentylméthyle, cyclopentyléthyle, cyclohexylméthyle, cyclohexyléthyle, norbornylméthyle et adamantylméthyle.
La liaison à la structure à laquelle il est rattaché se fait par le radical alkyle.

**«Hétérocycloalkyle »** désigne un système cyclique hydrocarboné non aromatique, ayant de 4 à 10 atomes de carbone, de préférence de 5 à 10 atomes de carbone, et de un à trois cycles, et comprenant de un à trois hétéroatomes choisi dans le groupe constitué par l'azote, l'oxygène et le soufre.
Les radicaux hétérocycloalkyles préférés contiennent de 5 à 7 atomes cycliques.
A titre d'exemples de radical hétérocycloalkyle, on peut citer les radicaux tétrahydrofuranyle, tétrahydrothiophényle, tétrahydropyrannyle, piperidinyle et 7-oxa-bicyclo-[2.2.1]-heptanyle.

**« Alkyle fluoré »** désigne un radical alkyle tel que défini précédemment substitué par un ou plusieurs atomes de fluor.
A titre d'exemples de radicaux alkyle fluoré, on peut citer les radicaux fluorométhyle, difluorométhyle, 2-fluoroéthyle, 2,2-difluoroéthyle et 2,2,2-trifluoroéthyle.

**« Alkyle perfluoré »** désigne un radical alkyle tel que défini précédemment dans lequel chaque atome d'hydrogène a été substitué par un atome de fluor.
A titre d'exemples de radicaux perfluoré, on peut citer les radicaux trifluorométhyle, et pentafluoroéthyle.

**« Halogène »** désigne un atome de fluor, de chlore, de brome ou d'iode. Les halogènes préférés sont les atomes de fluor et de chlore.

**« Sel pharmaceutiquement acceptable »** désigne les sels d'un composé d'intérêt qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Une liste de sels pharmaceutiquement acceptables est notamment publiée dans la revue de Berge et al. (J. Pharm. Sci. 1977, 66(1), 1-19).

Ainsi, un premier objet selon l'invention concerne de nouveaux composés antagonistes des récepteurs CXCR1 et CXCR2 aux chimiokines, ainsi que leurs sels et énantiomères, répondant à la formule générale (I) suivante: 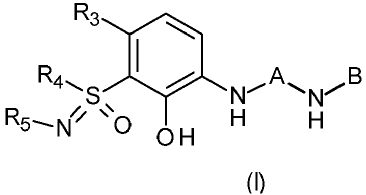 dans laquelle :
- A: represente 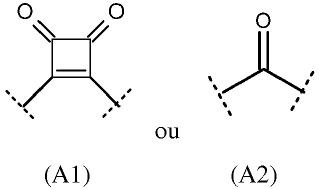
- B: represente 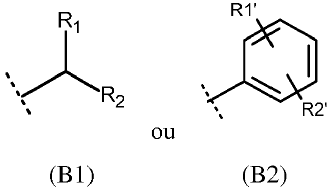
avec
R1' et R2', identiques ou différents, représentent un hydrogène, un halogène, un alkyle de C1 à C5 non substitué ou substitué par un ou plusieurs atomes de fluor, un alkoxy de C1 à C5, OCF3, OH, CN, NR11R12.
R1 et R2, identiques ou différents, représentent :
   - un hydrogène,
   - un alkyle de C1 à C5, non substitué ou substitué par un ou plusieurs groupes choisis parmi F, OH, OCH3 et NR11R12 ; R11 et R12 ayant la signification donnée ci-après, étant entendu que lorsque le radical alkyle de C1 à C5 n'est substitué que par un ou plusieurs atomes de fluor, il s'agit d'un radical alkyle fluoré ou d'un radical alkyle perfluoré de C1 à C5,
   - un alkyle de C1 à C5 dans lequel un atome de carbone est remplacé par un atome d'oxygène ou par un atome de soufre, ledit alkyle de C1 à C5 étant non substitué ou substitué par un ou plusieurs groupes choisis parmi F, OH, NR11R12, R11 et R12 ayant la signification donnée ci-après,
   - un cycloalkyle de C3 à C8
   - un alcyne de C2 à C5, non substitué ou substitué par un ou plusieurs groupes choisis parmi F, OH, phényle, NR11R12, R11 et R12 ayant la signification donnée ci-après,
   - un cycloalkyle répondant à l'une des formules (1), (2), (3), (4), (5) ou (6) suivantes dans lesquelles R5', X et X' ont les significations donnée ci-après : 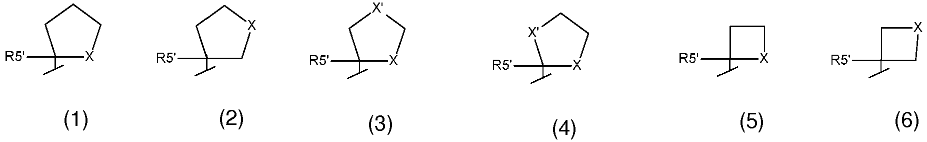
   - un cycle aromatique ou heteroaromatique sélectionné dans le groupe constitué par les cycles répondant aux formules (a) à (o) suivantes dans lesquelles R7, R7a, Y et Z ont les significations données ci-après: 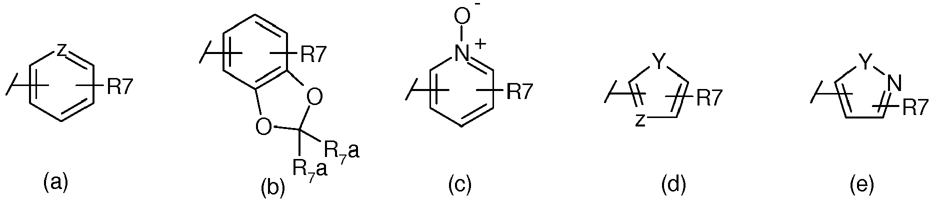 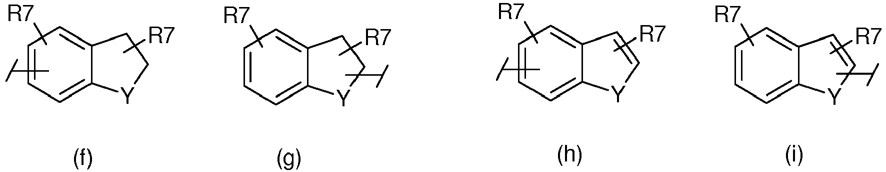 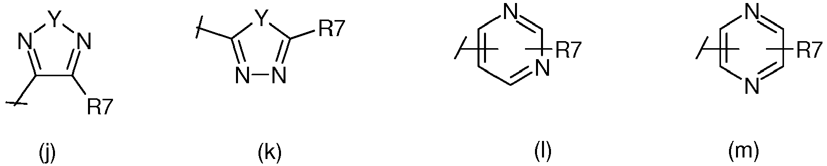 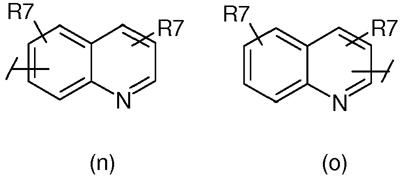
R7 peut être présent plusieurs fois sur un cycle, et au maximum, autant de fois qu'il y a d'atomes substituables. Les significations de chaque substituant R7 peuvent être identiques ou différentes.

- R3: représente un hydrogène, un halogène, un alkyle de C1 à C5, un alkoxy de C1 à C5, - CF3, -OCF3, -OH, -NO2, -CN
- R4 et R5,: identiques ou différents, représentent :
- un hydrogène,
- un alkyle de C1 à C8, non substitué ou substitué par un ou plusieurs groupes choisis parmi F, OH, NR11R12, R11 et R12 ayant la signification donnée ci-après,
- un alkyle de C1 à C8 dans lequel un atome de carbone est remplacé par un atome d'azote, par un atome d'oxygène ou par un atome de soufre, ledit alkyle de C1 à C8 étant non substitué ou substitué par un ou plusieurs groupes choisis parmi F, OH, NR11R12, R11 et R12 ayant la signification donnée ci-après,
- un cycloalkyle de C3 à C8,
- un cycloalkyle de C3 à C8 dont l'un des atomes de carbones est remplacé par un atome d'oxygène ou par un atome d'azote substitué par un radical R7a,
- un hétérocycloalkyle de 5 à 7 atomes cycliques,
- un cycloalkylalkyle, le cycloalkyle étant de C3 à C8 et l'alkyle de C1 à C8,
- un phényle,
- un phényle substitué par un radical R7,
- un heteroaryle,
- un arylalkyle, l'alkyle étant de C1-C5,
- un heteroarylalkyle, l'alkyle étant de C1-C5,
ou encore
- R4 et R5: représentent une chaine -(CH₂)ₘ- formant un cycle contenant de 5 à 8 atomes avec les atomes de soufre et d'azote auxquels ils sont respectivement rattachés, un des carbones du cycle étant optionnellement remplacé par un atome d'oxygène, de soufre ou par un atome d'azote substitué par un radical R8 ; m et R8 ayant les significations données ci-après,
- R5': représente un atome d'hydrogène, un fluor, un radical alkyle comportant de 1 à 5 atomes de carbone inclus ou un radical alkyle fluoré ou perfluoré comportant de 1 à 5 atomes de carbone,
- R6: représente un atome d'hydrogène, un radical -COOtBu ou un radical -COOBn,
- R7: représente un hydrogène, un alkyle de C1 à C3, un halogène, -CF3, -COR9, -OR9, - NR9R10, -NO2, -CN, -SO2R9, -S(O)R9, -S(=O)(=NR9)R10', -SO2NR9R10, - NR9SO2R10, -NR9COR10, -NR9CO2R10, -CONR9R10, ou -CO2R9,
- R7a: représente un hydrogène ou un alkyle de C1 à C5,
- R8: représente un hydrogène, -OH, -SO2R9, -COR9, -CO2R9, un aryle, un heteroaryle, un arylalkyle, un heteroarylalkyle, un alkyle, un cycloalkyle ou encore un cycloalkylalkyle,
- R9 et R10: sont identiques ou différents et sont indépendamment choisis dans le groupe constitué par un hydrogène, un aryle, un heteroaryle, un arylalkyle, un heteroarylalkyle, un alkyle, un cycloalkyle ou un cycloalkylalkyle,
ou encore
- R9 et R10: peuvent être liés entre eux lorsqu'ils sont portés par un même atome d'azote de façon à former un hétérocycle ayant entre 3 et 7 chainons et comportant un ou deux hétéroatomes choisis parmi l'oxygène , le soufre et l'azote en plus de l'atome d'azote auquel ils sont rattachés.
- R10': représente un aryle, un heteroaryle, un arylalkyle, un heteroarylalkyle, un alkyle, un cycloalkyle ou un cycloalkylalkyle,
- R11 et R12,: identiques ou différents, représentent un hydrogène, un alkyle de C1 à C5, un cycloalkyle de C3 à C6, une chaine -(CH₂)ₚ- formant un cycle contenant de 4 à 6 atomes avec l'atome d'azote auquel ils sont rattachés,
- X et X',: identiques ou différents, représentent un atome d'oxygène, un atome de soufre, ou un atome d'azote substitué par un radical R6,
- Y: représente un atome d'oxygène, un atome de soufre, ou un atome d'azote substitué par un radical R8,
- Z: représente un atome de carbone ou un atome d'azote,
m = 3, 4, 5, ou 6 et
p = 3, 4, ou 5.

Dans un mode de réalisation particulièrement préféré selon l'invention, les composés répondent à la formule générale (I) dans laquelle:
- A: représente 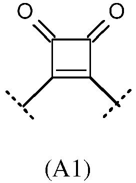
- B: représente 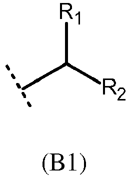
- R1: représente un hydrogène, un alkyle de C1 à C5, non substitué ou substitué par un ou plusieurs groupes choisis parmi F, OH et OCH3, un cycloalkyle de C3 à C8, un cycloalkyle répondant à la formule (1') suivante dans laquelle X a la signification donnée ci-après : 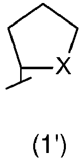
- R2: représente :
- un alkyle de C1 à C5, non substitué ou substitué par un ou plusieurs groupes choisis parmi F, étant entendu que lorsque le radical alkyle de C1 à C5 n'est substitué que par un ou plusieurs atomes de fluor, il s'agit d'un radical alkyle fluoré ou d'un radical alkyle perfluoré de C1 à C5,
- un alkyle de C1 à C5 dans lequel un atome de carbone est remplacé par un atome d'oxygène,
- un alcyne de C2 à C5, non substitué ou substitué par un ou plusieurs groupes choisis parmi le fluor et le phényle,
- un cycle aromatique ou heteroaromatique sélectionné dans le groupe constitué par les cycles répondant aux formules (a), (b1) et (d1) suivantes dans lesquelles R7 et Z ont les significations données ci-après: 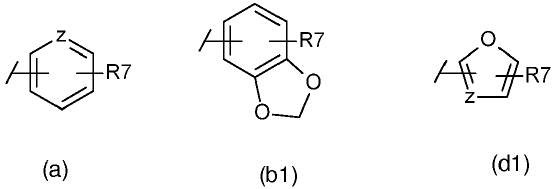 R7 peut être présent plusieurs fois sur un cycle, et au maximum, autant de fois qu'il y a d'atomes substituables. Les significations de chaque substituant R7 peuvent être identiques ou différentes,
- R3: représente un hydrogène ou un chlore,
- R4 et R5,: identiques ou différents, représentent :
- un hydrogène,
- un alkyle de C1 à C3, non substitué ou substitué par un groupe NR11R12, R11 et R12 ayant la signification donnée ci-après,
- un alkyle de C1 à C8 dans lequel un atome de carbone est remplacé par un atome d'oxygène,
- un pyridyle,
- un piperidinyle,
- R7: représente un hydrogène, un alkyle de C1 à C3 ou un fluor,
- X: représente un atome de soufre, et
- Z: représente un atome de carbone ou un atome d'azote.

Dans un mode de réalisation plus particulièrement préféré selon l'invention, les composés répondent à la formule générale (I) dans laquelle:
- A: représente 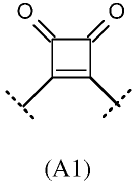
- B: représente 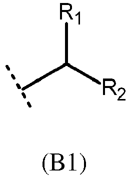
- R1: représente un hydrogène, un alkyle de C1 à C5, un cycloalkyle de C3 à C8, un cycloalkyle répondant à la formule (1') suivante dans laquelle X a la signification donnée ci-après : 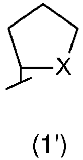
- R2: représente :
- un alkyle de C1 à C5, non substitué ou substitué par un ou plusieurs groupes choisis parmi F, étant entendu que lorsque le radical alkyle de C1 à C5 n'est substitué que par un ou plusieurs atomes de fluor, il s'agit d'un radical alkyle fluoré ou d'un radical alkyle perfluoré de C1 à C5,
- un alkyle de C1 à C5 dans lequel un atome de carbone est remplacé par un atome d'oxygène,
- un alcyne de C2 à C5, non substitué ou substitué par un ou plusieurs groupes choisis parmi le fluor et le phényle,
- un cycle aromatique ou heteroaromatique sélectionné dans le groupe constitué par les cycles répondant aux formules (a), (b1) et (d1) suivantes dans lesquelles R7 et Z ont les significations données ci-après: 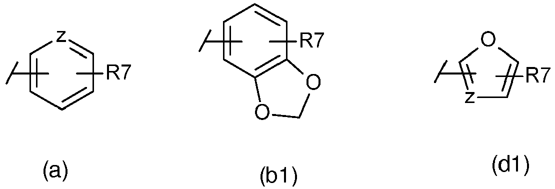 R7 peut être présent plusieurs fois sur un cycle, et au maximum, autant de fois qu'il y a d'atomes substituables. Les significations de chaque substituant R7 peuvent être identiques ou différentes,
- R3: représente un hydrogène ou un chlore,
- R4 et R5,: identiques ou différents, représentent un hydrogène, un alkyle de C1 à C3, un alkyle de C1 à C8 dans lequel un atome de carbone est remplacé par un atome d'oxygène,
- R7: représente un hydrogène, un alkyle de C1 à C3 ou un fluor,
- X: représente un atome de soufre, et
- Z: représente un atome de carbone ou un atome d'azote.

Les composés répondant au mode de réalisation plus particulièrement préféré tels que décrits ci-dessus présentent des activités antagonistes aux récepteurs CXCR1 inférieures à 400 nM et des activités antagonistes aux récepteurs CXCR2 inférieures à 100 nM.

Ces composés, en série sulfoximine, présentent une activité supérieure à celle de leurs analogues structuralement les plus proches pris dans l'art antérieur en série sulfone comme illustré ci-dessous à titre d'exemple :

**Tableau 1**

| | | |
|---|---|---|
| | 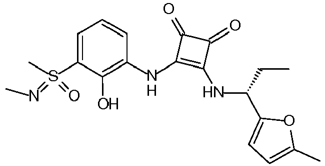 | 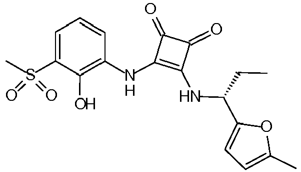 |

| | Exemple 16 | WO02/083624 |
|---|---|---|
| CXCR1 IC50 (nM) | 36 | 418 |
| CXCR2 IC50 (nM) | 18 | 103 |
| Migration des neutrophiles h IC50 (nM) | 356 | 3090 |

Dans le mode de réalisation le plus préféré selon l'invention, les composés répondent à la formule générale (I) précitée dans laquelle:
- A: représente 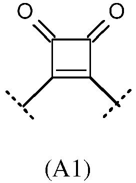
- B: représente 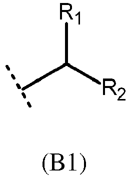
- R1: représente un alkyle de C1 à C5,
- R2: représente :
- un alkyle de C1 à C5,
- un alcyne de C2 à C5, substitué par un ou plusieurs groupes choisis parmi le fluor et le phényle,
- un cycle aromatique ou heteroaromatique sélectionné dans le groupe constitué par les cycles répondant aux formules (a), (b1) et (d1) suivantes dans lesquelles R7 et Z ont les significations données ci-après: 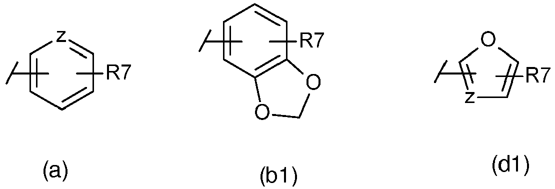 R7 peut être présent plusieurs fois sur un cycle, et au maximum, autant de fois qu'il y a d'atomes substituables. Les significations de chaque substituant R7 peuvent être identiques ou différentes,
- R3: représente un hydrogène ou un chlore,
- R4 et R5,: identiques ou différents, représentent un hydrogène ou un alkyle de C1 à C3,
- R7: représente un hydrogène, un alkyle de C1 à C3 ou un fluor, et
- Z: représente un atome de carbone ou un atome d'azote.

Les composés répondant au mode de réalisation le plus préféré tels que décrits ci-dessus présentent des activités antagonistes aux récepteurs CXCR1 inférieures à 50 nM et des activités antagonistes aux récepteurs CXCR2 inférieures à 20 nM.

Ces composés, en série sulfoximine, présentent une activité supérieure à celle de leurs analogues les plus proches pris dans l'art antérieur en série sulfone comme illustré ci-dessous à titre d'exemple :

**Tableau 2**

| | 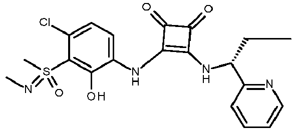 | 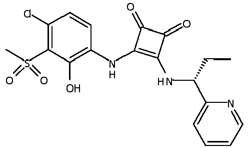 |
|---|---|---|
| | Exemple 12 | WO02/083624 |
| CXCR1 IC50 (nM) | 28 | 155 |
| CXCR2 IC50 (nM) | 16 | 66 |
| Migration des neutrophiles h IC50 (nM) | 82 | 831 |

Dans un autre mode de réalisation selon l'invention, les composés répondent à la formule générale (I) dans laquelle:
- A: représente 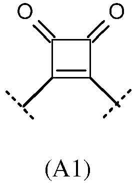
- B: représente 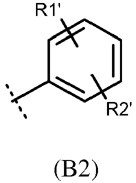
- R1' et R2',: identiques ou différents, représentent un hydrogène, un chlore ou un fluor,
- R3: représente un hydrogène ou un chlore,
- R4 et R5,: identiques ou différents, représentent un hydrogène, un alkyle de C1 à C3,

Parmi les composés plus particulièrement préférés, on peut citer par exemple ceux choisis dans la liste comprenant:
Composé 1: 3-(4-Chloro-2-hydroxy-3-methanesulfoxymin-phenylamino)-4-[(R)-1-(5-methyl-furan-2-yl)-propylamino]-cyclobut-3-ene-1,2-dione
Composé 2: 3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione
Composé 3: 3-{4-Chloro-2-hydroxy-3-methane-[(N- pyridin-4-yl)-sulfoximin]-phenylamino]-4-[(R)-1-(5-methyl-furan-2-yl)-propylamino]-cyclobut-3-ene-1,2-dione
Composé 4: 3-{4-Chloro-2-hydroxy-3-methane-[(N-pyridin-4-yl)-sulfoximin]-phenylamino}-4-(1-Ethyl-propylamino)-cyclobut-3-ene-1,2-dione
Composé 5: 3-{4-Chloro-2-hydroxy-3-methane-[(N-methyl)-sulfoximin]-phenylamino}-4-{[(S)-(5-methyl-furan-2-yl)-(R)-tetrahydro-thiophen-2-yl-methyl]-amino}-cyclobut-3-ene-1,2-dione
Composé 6: 1-(2-chloro-3-fluoro-phenyl)-3-(4-chloro-2-hydroxy-3-methanesulfoximine-phenyl)-urea
Composé 7: 1-(2-Chloro-3-fluoro-phenyl)-3-{4-chloro-2-hydroxy-3-methanes-[(N-methyl)-sulfoximin]-phenyl}-urea
Composé 8: 3-[2-hydroxy-3-methane-[(N-methyl)-sulfoximin]-phenylamino]-4-[(R)-1-(5-methyl-furan-2-yl)-propylamino]-cyclobut-3-ene-1,2-dione
Composé 9: 3-[4-chloro-3-[N-(2-dimethylaminoethyl)-S-methyl-sulfonimidoyl]-2-hydroxy-anilino]-4-ethoxy-cyclobut-3-ene-1,2-dione
Composé 10: 3-[4-chloro-3-[N-(2-dimethylaminoethyl)-S-methyl-sulfonimidoyl]-2-hydroxy-anilino]-4-(1-ethylpropylamino)cyclobut-3-ene-1,2-dione
Composé 11:3-[4-chloro-2-hydroxy-3-[N-(2-methoxyethyl)-S-methyl-sulfonimidoyl]anilino]-4-[[(1R)-1-(5-methyl-2-furyl)propyl]amino]cyclobut-3-ene-1,2-dione
Composé 12: (-)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione
Composé 13: (+)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione
Composé 14: (-)-3-{2-Hydroxy-3-methane-[(N-methyl)-sulfoximin]-phenylamino}-4-[(R)-1-(pyridin-2-yl)-propylamino]-cyclobut-3-ene-1,2-dione
Composé 15: (-)-3-{2-Hydroxy-3-methane-[(N-methyl)-sulfoximin]-phenylamino}-4-[(R)-1-(pyridin-2-yl)-propylamino]-cyclobut-3-ene-1,2-dione
Composé 16: (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione
Composé 17: (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione
Composé 18: (-)-3-(2-Hydroxy-3-methane-[(N-methyl)-sulfoxim]-phenylamino-[4-(1-ethyl-propylamino]-cyclobut-3-ene-1,2-dione
Composé 19: (+)-3-(2-Hydroxy-3-methane-[(N-methyl)-sulfoxim]-phenylamino-[4-(1-ethyl-propylamino]-cyclobut-3-ene-1,2-dione
Composé 20: (+)-3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione

Un second objet selon l'invention concerne une composition pharmaceutique comprenant une quantité efficace d'un composé répondant à la formule générale (I) tel que décrit ci-dessus en association avec un solvant ou un support pharmaceutiquement acceptable.

Un troisième objet selon l'invention concerne les composés répondant à la formule générale (I), ainsi que leurs sels et énantiomères, ou encore une composition pharmaceutique comprenant une quantité efficace d'un composé répondant à la formule générale (I), d'un de ses sels ou énantiomères pour leur utilisation en tant que médicament.

Un quatrième objet selon l'invention concerne les composés répondant à la formule générale (I), ainsi que leurs sels et énantiomères ou encore une composition pharmaceutique comprenant une quantité efficace d'un composé répondant à la formule générale (I), d'un de ses sels ou énantiomères pour leur utilisation dans le traitement de maladies médiées par les α-chimiokines telles que définies dans la présente demande.

A titre d'exemples de maladies médiées par les α- chimiokines, on peut citer les dermatoses neutrophiliques, notamment le psoriasis, les dermatites atopiques, l'acné, la rosacée, l'asthme, les maladies pulmonaires obstructives chroniques, les maladies respiratoires des adultes, l'arthrite, les maladies intestinales inflammatoires, la maladie de Crohn, le rejet de greffe, la mucoviscidose et les cancers cutanés.

Par dermatoses neutrophiliques, on entend dans son sens le plus large, le syndrome de Sweet, « eccrines hydranite », le syndrome SAPHO, le syndrome de Sneddon Wilkinson, le pyoderma gangrenosum, l'érythème elevatum duitinum, le psoriasis, le psoriasis vulgaire, le psoriasis pustuleux, la pustulose palmo-plantaire, la pustulose exanthématique (PEAG), la pustulose vascularite, l'acro-pustulose de l'enfant, la maladie de Behcet, ainsi que certains maladies bulleuses comme l'herpès dérivés sous forme de dermatite, la dermatose neutrophilique à IgA, IgA intra-épiderme pustilosis, la pemphigoïde bulleuse, le pemphigus à IgA, la vascularite, le syndrome de Leroy Reiter Fiellinger,la pustulose du cuir chevelu, l'acrodermatite continue d' Hallopeau et les dermatoses liées à l'angio-immunoblastique lymphodénopathie, avec dysmielopoësis induite par le cyclophosphamide, avec des p-ANCA anticorps.

Dans un mode de réalisation préféré selon l'invention, le composé ou la composition pharmaceutique précitée est utilisé dans le traitement de maladies dermatologiques telles que les dermatoses neutrophiliques, notamment le psoriasis, les dermatites atopiques, l'acné et la rosacée.

Il est également décrit l'utilisation d'un composé répondant à la formule générale (I), ainsi que leurs sels et énantiomères ou encore l'utilisation d'une composition pharmaceutique comprenant une quantité efficace d'un composé répondant à la formule générale (I), d'un de ses sels et de ses énantiomères pour la préparation d'un médicament pour le traitement des maladies du groupe comprenant les dermatoses neutrophiliques, notamment le psoriasis, les dermatites atopiques, l'acné, la rosacée, l'asthme, les maladies pulmonaires obstructives chroniques, les maladies respiratoires des adultes, l'arthrite, les maladies intestinales inflammatoires, la maladie de Crohn et les cancers cutanés.

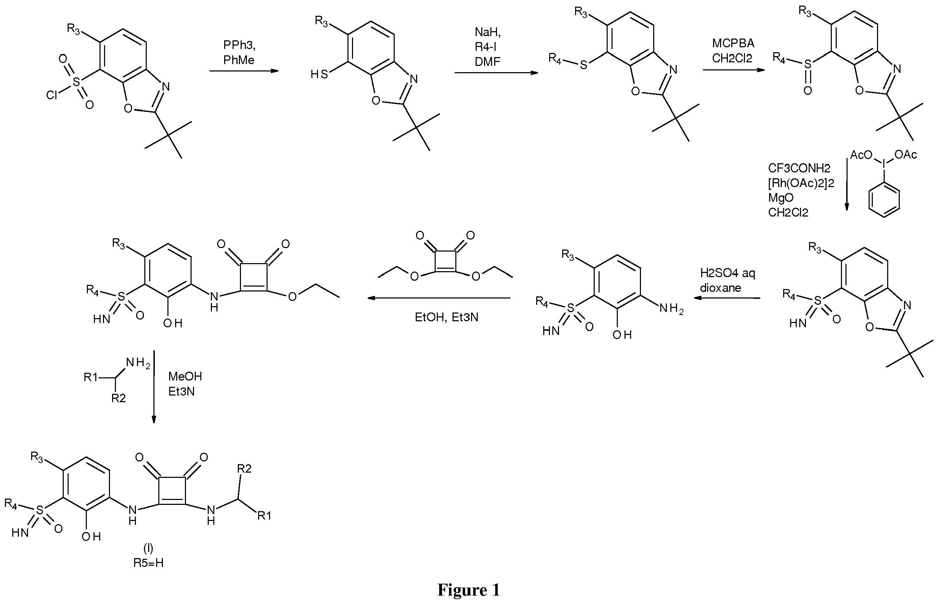

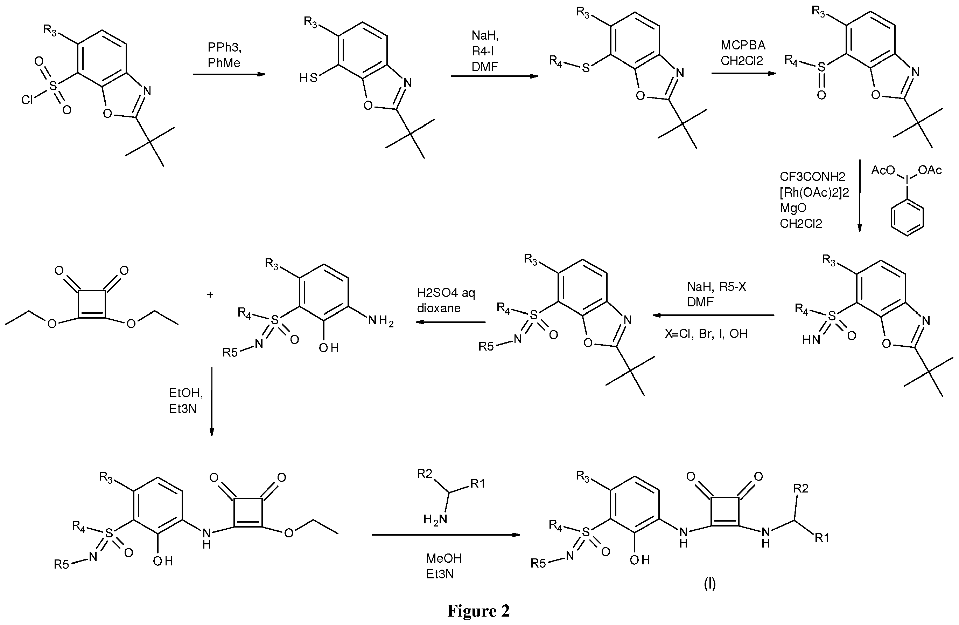

Les composés de formule générale (I) de la présente invention sont préparés suivant une des deux voies de synthèse telles qu'elles ressortent des schémas de synthèse indiqués aux figures 1 et 2. L'homme du métier pourra aisément déduire les conditions expérimentales requises pour chacune de ces deux voies de synthèse en se référant à celles utilisées pour chaque voie de synthèse telles qu'elles ressortent des exemples de préparation des composés de formule (I) illustrés ci-après de façon non limitative.

A titre d'illustration, les composés suivants, répondant à la formule générale (I) de la présente invention, ont été préparés en suivant l'un des deux schémas présentés ci-dessus et aux figures 1 et 2.

### PREPARATION DES COMPOSES DE FORMULE (I)

### EXEMPLE 1

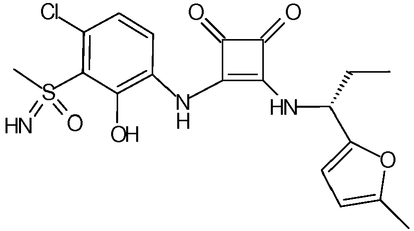

### 3-(4-Chloro-2-hydroxy-3-methanesulfoxymin-phenylamino)-4-[(R)-1-(5-methyl-furan-2-yl)-propylamino]-cyclobut-3-ene-1,2-dione

A une suspension de 3-(4-chloro-2-hydroxy-3-methanesulfoximin-phenylamino)-4-ethoxy-cyclobut-3-ene-1,2-dione (0.19 g; 0.51 mmol) dans du méthanol (8 ml) sont rajoutées une solution de chlorhydrate de (R)-1-(5-methyl-furan-2-yl)-propylane (0.11 g; 0.62 mmol) dans du méthanol (1 ml) et de la triethylamine (85 µl; 0.62 mmol). Au bout de trois jours, le milieu réactionnel est partiellement concentré. De l'acétate d'éthyle puis une solution aqueuse à 1 M de dihydrogénophosphate de sodium sont rajoutés. La phase organique est à nouveau lavée avec une solution aqueuse à 1 M de dihydrogénophosphate de sodium. Le solide obtenu est chromatographié sur gel de silice (éluant heptane/acétone, de 20% à 70% d'acétone). 3-(4-Chloro-2-hydroxy-3-methanesulfoxymin-phenylamino)-4-[(R)-1-(5-methyl-furan-2-yl)-propylamino]-cyclobut-3-ene-1,2-dione (0.12 g; 50%) est obtenu. Le point de fusion 192°C. MS(ES+) *m*/*z* 438 (MH+).
1H NMR (400 MHz, DMSO-d6): δ 0.92 (td, J = 7.3, 1.5 Hz, 3H); 1.90-1.92 (m, 2H); 2.27 (s, 3H); 3.64 (s, 3H); 5.13 (q, J = 7.8 Hz, 1H); 6.06 (dd, J = 3.1, 1.3 Hz, 1H); 6.27 (t, J = 2.6 Hz, 1H); 7.02 (d, J = 8.7 Hz, 1H); 8.03 (dd, J = 8.7, 2.0 Hz, 1H); 8.76 (dd, J = 9.0, 3.9 Hz, 1H); 9.36 (d, J = 3.7 Hz, 1H); 11.72 (s, 2H).

### 3-(4-chloro-2-hydroxy-3-methanesulfoximin-phenylamino)-4-ethoxy-cyclobut-3-ene-1,2-dione

A une solution de 6-amino-3-chloro-2-methanesulfoximin-phenol (0.52 g; 2.12 mmol) dans de l'ethanol (35 ml) est rajouté du 3,4-diethoxy-3-cyclobutene-1,2-dione (0.63 ml; 4.24 mmol). Le milieu réactionnel est chauffé pendant 3 jours à 60°C puis est concentré. L'huile obtenue est chromatographiée sur gel de silice (éluant heptane/acétone, de 10% à 80% d'acétone). 3-(4-Chloro-2-hydroxy-3-methanesulfoximin-phenylamino)-4-ethoxy-cyclobut-3-ene-1,2-dione (0.39 g; 49%) est obtenu. MS(ES+) *m*/*z* 345/347 (MH+).

### 6-amino-3-chloro-2-methanesulfoximin-phenol

A une solution de 2-tert-butyl-6-chloro-7-methanesulfoximin-benzooxazole (0.80 g; 2.79 mmol) dans du 1,4-dioxanne (4 ml) sont rajoutés de l'eau (1 ml) et d'acide sulfurique (0.95 ml; 17.77 mmol). Le milieu réactionnel est ensuite chauffé à 100°C. Au bout d'une heure 40 minutes, le milieu réactionnel est refroidis et hydrolysé avec une solution de soude 1N et de l'eau est rajoutée (jusqu'à pH=6-7). Le mélange est extrait à l'acétate d'éthyle. La phase organique est séchée sur sulfate de sodium anhydre, filtrée et concentrée. 6-Amino-3-chloro-2-methanesulfoximin-phenol (0.55 g; 80 %) est obtenu. MS(ES+) *m*/*z* 221/223 (MH+).

### 2-tert-Butyl-6-chloro-7-methanesulfoximin-benzooxazole

A une suspension de 2-tert-butyl-6-chloro-7-methanesulfinyl-benzooxazole (11.11 g; 40.88 mmol) de 2,2,2-trifluoroacetamide (9.24 g; 81.76 mmol), d'oxide de magnesium (6.59 g; 163.52 mmol) dans du dichloromethane (400 ml) dégazé environ 15 minutes sous azote sont rajoutés du rhodium(II)acetate dimer (1.45 g; 3.27 mmol) et du iodobenzene diacetate (20.15 g; 62.55 mmol). Au bout de 17 heures, du 2,2,2-trifluoroacetamide (2.50 g; 22.12 mmol), du iodobenzene diacétate (3.48 g; 10.80 mmol) et du rhodium(II) acétate dimer (0.69 g; 1.56 mmol) sont rajoutés. Au bout de 20 heures, le milieu réactionnel est chauffé au reflux. Au bout de 45 heures, le milieu réactionnel est filtré sur célite et est concentré. Le résidu est repris dans du méthanol (400 ml) et du carbonate de potassium (28.25 g; 204.40 mmol) est rajouté. Au bout d'une heure, le milieu réactionnel est concentré. Le résidu est repris dans de l'acétate d'éthyle et est lavé à l'eau. La phase aqueuse est à nouveau extraite à l'acétate d'éthyle. Les phases organiques sont réunies, séchées sur sulfate de sodium anhydre, filtrées et concentrées. L'huile obtenue est chromatographiée sur gel de silice (éluant heptane/acétate d'éthyle, de 5% à 80% d'acétate d'éthyle). 2-tert-Butyl-6-chloro-7-methanesulfoximin-benzooxazole (1.76 g; 15 %) est obtenu. MS(ES+) *m*/*z* 287/289 (MH+).

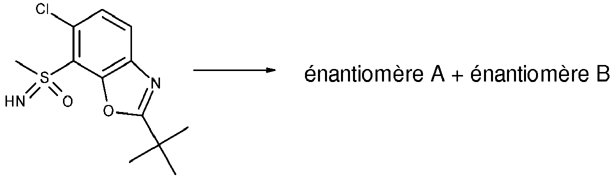

### (-)-2-tert-Butyl-6-chloro-7-methanesulfoximin-benzooxazole (énantiomère A) (+)-2-tert-Butyl-6-chloro-7-methanesulfoximin-benzooxazole (énantiomère B)

2-tert-Butyl-6-chloro-7-methanesulfoximin-benzooxazole 2.5 g a été séparé en énantiomères par la méthode HPLC chiral sur la colonne CHIRALCEL OJ 20 µm LC50 avec le solvant heptane/éthanol 90:10 comme l'éluant. La séparation a donné l'énantiomère A (1.35 g, temps de rétention 11.71 min, [α]_{D} = -2.4° (c = 10 g/L, EtOH)) et l'énantiomère B (1.37 g, temps de rétention 23.10 min, [α]_{D} = +3.5° (c = 10 g/L, EtOH)).

### 2-tert-butyl-6-chloro-7-methanesulfinyl-benzooxazole

A une solution de 2-tert-butyl-6-chloro-7-methylsulfanyl-benzooxazole (9.78 g; 38.24 mmol) dans du dichlorométhane (200 ml) sous azote est rajouté par portion de l'acide 3-chloroperbenzoïque (9 g; 40.15 mmol). Au bout de 24 heures, le milieu réactionnel est hydrolysé avec de la soude 2N. La phase organique est lavée une seconde fois avec une solution de soude 2N. La phase organique est séchée sur sulfate de sodium anhydre, filtrée et concentrée. 2-tert-Butyl-6-chloro-7-methanesulfinyl-benzooxazole (11.9 g; 100 %) est obtenu. MS(ES+) *m*/*z* 272/274 (MH+).

### 2-tert-Butyl-6-chloro-7-methylsulfanyl-benzooxazole

A une suspension d'hydride de sodium à 60% dans l'huile (2.20 g; 55.00 mmol) dans du tetrahydrofuranne (135 ml), sous azote, dans un bain à 0°C sont rajoutés, goutte à goutte, une solution de 2-tert-butyl-6-chloro-benzooxazole-7-thiol (12 g; 49.64 mmol) dans du tetrahydrofuranne (60 ml) puis 15 minutes plus tard du iodométhane (9.5 ml; 152.60 mmol). Le milieu réactionnel est ensuite remis à température ambiante. Au bout de 16 heures, une solution aqueuse à 1 M de dihydrogénophosphate de sodium est rajoutée ainsi que de l'acétate d'éthyle. La phase organique est séchée sur sulfate de sodium anhydre, filtrée et concentrée. L'huile obtenue est chromatographiée sur gel de silice (l'éluant heptane/acétate d'éthyle, de 0% à 10% d'acétate d'éthyle). 2-tert-Butyl-6-chloro-7-methylsulfanyl-benzooxazole (10.85 g; 85%) est obtenu. MS(ES+) *m*/*z* 256/258 (MH+).

### 2-tert-Butyl-6-chloro-benzooxazole-7-thiol

A une solution de 2-tert-butyl-6-chlorobenzoxazole-7-sulfonyl chloride (15.01 g; 48.70 mmol) sous azote dans du toluene (250 ml) est rajoutée une solution de triphenylphosphine (38.32 g; 146.11 mmol) dans du toluene (125 ml) (la réaction est exothermique). Au bout de 40 minutes, le milieu réactionnel est hydrolysé avec de l'eau (250 ml) et est laissé agiter pendant une heure. La phase organique est ensuite extraite deux fois avec une solution de soude 1N (2 x 125 ml). Les phases aqueuses basiques sont réunies et lavées deux fois au toluène (2 x 125 ml). La phase aqueuse est ensuite acidifiée jusqu'à pH = 1 avec une solution aqueuse d'acide chlorhydrique 2N puis est extraite deux fois au dichlorométhane (2 x 150 ml). Les phases organiques sont réunies, séchées sur sulfate de sodium anhydre, filtrées et concentrées. 2-tert-Butyl-6-chloro-benzooxazole-7-thiol (11.37 g; 96%) est obtenu. MS(ES+) *m*/*z* 342/344 (MH+).

### EXEMPLE 2

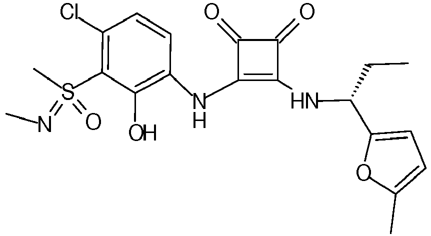

### 3-{4-Chloro-2-hydroxy-3-methane-[(N-methyl)-sulfoximin]-phenylamino}-4-[(R)-1-(5-methyl-furan-2-yl)-propylamino[-cyclobut-3-ene-1,2-dione

A un mélange de 3-(4-chloro-2-hydroxy-3-methane-[(N-methyl)-sulfoximin]-phenylamino)-4-ethoxy-cyclobut-3-ene-1,2-dione (0.16 g; 0.45 mmol) dans du méthanol (3 ml) sont rajoutées une solution de chlorhydrate de (R)-1-(5-methyl-furan-2-yl)-propylane (90 mg; 0.51 mmol) dans du méthanol (1 ml) et de la triethylamine (70 µl; 0.50 mmol). Le milieu réactionnel est ensuite chauffé à 50°C. Au bout de 2 heures, de l'acétate d'éthyle est rajouté et le milieu réactionnel est lavé deux fois avec une solution aqueuse à 1 M de dihydrogénophosphate de sodium. La phase organique est séchée sur sulfate de sodium anhydre, filtrée et concentrée. Le solide obtenu est mis à sécher sous vide à l'étuve à 50°C. 3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione (0.18 g; 88%) est obtenu de point de fusion 131°C. MS(ES+) *m*/*z* 452 (MH+)
1H NMR (400 MHz, DMSO): δ 0.92 (td, J = 7.3, 1.4 Hz, 3H); 1.90-1.92 (m, 2H); 2.27 (s, 3H); 2.91 (s, 3H); 3.70 (s, 3H); 5.13 (q, J = 7.7 Hz, 1H); 6.06 (dd, J = 3.0, 1.2 Hz, 1H); 6.26 (t, J = 2.6 Hz, 1H); 6.98 (d, J = 8.7 Hz, 1H); 8.05 (dd, J = 8.7, 2.2 Hz, 1H); 8.78 (dd, J = 9.0, 3.6 Hz, 1H); 9.36 (d, J = 3.7 Hz, 1H).

### 3-(4-chloro-2-hydroxy-3-methane-[(N-methyl)-sulfoximin]-phenylamino)-4-ethoxy-cyclobut-3-ene-1,2-dione

A une solution de 6-amino-3-chloro-2-methane-[(N-methyl)-sulfoximin]-phenol (0.39 g; 1.33 mmol) dans de l'ethanol (35 ml) est rajouté du 3,4-diethoxy-3-cyclobutene-1,2-dione (0.50 ml; 3.38 mmol). Le milieu réactionnel est chauffé à 60°C. Au bout de trois jours, le milieu réactionnel est concentré. L'huile obtenue est chromatographiée sur gel de silice (l'éluant heptane/acétone, de 20% à 50% d'acétone). 3-(4-Chloro-2-hydroxy-3-methane-[(N-methyl)-sulfoximin]-phenylamino)-4-ethoxy-cyclobut-3-ene-1,2-dione (0.32 g; 67%) est obtenu. MS(ES+) *m*/*z* 359/361 (MH+).

### 6-amino-3-chloro-2-methane-[(N-methyl)-sulfoximin]-phenol

A une solution de 2-tert-butyl-6-chloro-7-methane-N-methyl-sulfoximin-benzooxazole (0.50 g; 1.66 mmol) dans du 1,4-dioxanne (2.5 ml) sont rajoutés de l'eau (0.60 ml) et de l'acide sulfurique concentré gout à gout (0.57 ml; 10.66 mmol). Le milieu réactionnel est chauffé à 100°C pendant 1h30min, puis 2 heures à 70°C puis remis à 100°C. Au bout de 4 heures de réaction, de l'acide sulfurique concentré (0.20 ml; 3.74 mmol) est rajouté. Au bout de 7 heures de réaction de l'acide sulfurique concentré (0.40 ml; 7.48 mmol) est à nouveau rajouté. Au bout de 8 heures, le milieu réactionnel est hydrolysé avec de l'eau et une solution aqueuse de soude 1N est rajoutée (jusqu'à pH=7). Le mélange est extrait à l'acétate d'éthyle. La phase organique est séchée sur sulfate de sodium anhydre, filtrée et concentrée. 6-Amino-3-chloro-2-methane-[(N-methyl)-sulfoximin]-phenol (0.39 g; 80%) est obtenu. MS(ES+) *m*/*z* 235/237 (MH+).

### 2-tert-butyl-6-chloro-7-methane-N-methyl-sulfoximin-benzooxazole

A une suspension d'hydride de sodium à 60% dans l'huile (0.12 g; 3.00 mmol) dans du N,N-dimethylformamide (15 ml) à 0°C sous azote est rajoutée une solution de 2-tert-butyl-6-chloro-7-methanesulfoximin-benzooxazole (0.74 g; 2.58 mmol) dans du N,N-dimethylformamide (15 ml). 20 minutes plus tard, du iodométhane (0.32 ml; 5.14 mmol) est rajouté. Le milieu réactionnel est ensuite remis à température ambiante. Au bout de 3 heures 30 minutes, le milieu réactionnel est hydrolysé avec de l'eau et est extrait à l'acétate d'éthyle. La phase organique est lavée une autre fois à l'eau, séchée sur sulfate de sodium anhydre, filtrée et concentrée. L'huile obtenue est chromatographiée sur gel de silice (l'éluant heptane/acétate d'éthyle, de 30% à 80% d'acétate d'éthyle). 2-tert-Butyl-6-chloro-7-methane-N-methyl-sulfoximin-benzooxazole (0.51 g; 65%) est obtenu. MS(ES+) *m*/*z* 301/303 (MH+).

### EXEMPLE 3

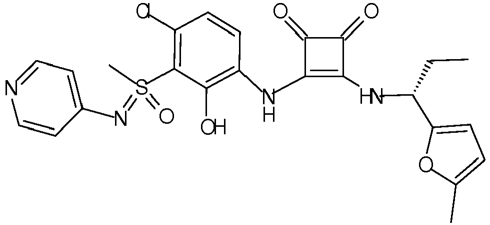

### 3-{4-Chloro-2-hydroxy-3-methane-[(N-pyridin-4-yl)-sulfoximin]-phenylamino}-4-[(R)-1-(5-methyl-furan-2-yl)-propylamino[-cyclobut-3-ene-1,2-dione

A une suspensione de 3-(4-chloro-2-hydroxy-3-methane-[(N-pyridin-4-yl)-sulfoximin]-phenylamino)-4-ethoxy-cyclobut-3-ene-1,2-dione (114 mg; 0.27 mmol) et de chlorhydrate de (R)-1-(5-methyl-furan-2-yl)-propylane (54.59 mg; 0.31 mmol) dans du méthanol (3 ml) est ajoutée de la triethylamine (0.04 ml; 0.31 mmol). Le milieu réactionnel est chauffé à 60°C pendant 24 heures. Le milieu réactionnel refroidi est dilué avec au dichlorométhane et lavé deux fois avec une solution aqueuse à 1 M de dihydrogénophosphate de sodium. La phase organique est séchée sur sulfate de magnésium anhydre, filtrée et concentrée. Le solide obtenu est repris avec du méthanol et cristallisé à l'ether. Le composé est chromatographié sur gel de silice, éluant dichlorométhane méthanol 95/05. On obtient 3-{4-chloro-2-hydroxy-3-methane-[(N-pyridin-4-yl)-sulfoximin]-phenylamino}-4-[(R)-1-(5-methyl-furan-2-yl)-propylamino]-cyclobut-3-ene-1,2-dione (70 mg; 48%). MS(ES+) *m*/*z* 515 (MH+).
1H NMR (400 MHz, DMSO): δ (ppm) 0.87-0.92 (m, 3H); 1.81 (dt, J = 14.0, 7.0 Hz, 1H); 1.89-1.94 (m, 1H); 2.25 (d, J = 6.7 Hz, 3H); 3.79 (s, 3H); 5.11 (q, J = 7.6 Hz, 1H); 6.00-6.03 (m, 1H); 6.20 (t, J = 4.3 Hz, 2H); 6.98 (dd, J = 7.0, 2.2 Hz, 2H); 7.78 (d, J = 8.2 Hz, 1H); 8.23 (dd, J = 6.7, 4.6 Hz, 2H); 8.86 (d, J = 9.0 Hz, 1H); 9.41 (s, 1H).

### 3-(4-Chloro-2-hydroxy-3-methane-[(N-pyridin-4-yl)-sulfoximin]-phenylamino)-4-ethoxy-cyclobut-3-ene-1,2-dione

A une solution de 6-amino-3-chloro-2-methane-[(N-methyl)-sulfoximin]-phenol (222 mg; 0.75 mmol) dans de l'ethanol (20 ml) est rajouté du 3,4-diethoxy-3-cyclobutene-1,2-dione (0.30 ml; 2.01 mmol). Le milieu réactionnel est chauffé à 60°C pendant la nuit. Le milieu réactionnel est concentré et repris dans de l'acétate d'éthyle puis l'ether. On obtient 3-(4-chloro-2-hydroxy-3-methane-[(N-pyridin-4-yl)-sulfoximin]-phenylamino)-4-ethoxy-cyclobut-3-ene-1,2-dione (228 mg; 72%). MS(ES+) *m*/*z* 422 (MH+)

### 6-Amino-3-chloro-2-methane-[(N-pyridin-4-yl)-sulfoximin]-phenol

Sur 2-tert-butyl-6-chloro-7-(N-pyridin-4-yl)sulfoximin-benzooxazole (320 mg; 0.88 mmol) en solution dans 1,4-dioxanne (5 ml) est ajouté goutte à goutte de l'acide sulfurique (0.87 ml; 16.20 mmol) dilué dans l'eau (1.28 ml) à température ambiante. Le milieu réactionnel est agité à 100°C pendant 4h. Le pH du milieu est ramené à pH=7-8 avec de la soude 1N puis extraction à l'acétate d'éthyle. La phase organique est séchée, filtrée et concentré. On obtient 6-amino-3-chloro-2-methane-[(N-pyridin-4-yl)-sulfoximin]-phenol (222 mg; 84%). MS(ES+) *m*/*z* 298 (MH+).

### 2-tert-Butyl-6-chloro-7-(N-pyridin-4-yl)sulfoximin-benzooxazole

Un mélange de chlorhydrate de 4-bromopyridine (848 mg; 4.36 mmol), de 2-tert-butyl-6-chloro-benzooxazole-7-sulfoximine (1.00 g; 3.49 mmol), de l'acétate de palladium (39.14 mg; 0.17 mmol), de rac-2,2'bis(diphenylphosphino)-1,1'binaphtyl 97% (21.71 mg; 0.03 mmol) et de carbonate de césium (2.84 g; 8.72 mmol) dans du toluene (12.5 ml) (dégazé) est chauffé à 110°C pendant 40 heures. Le milieu réactionnel est repris à l'eau, la phase organique est extraite trois fois à l'acétate d'éthyle, séchée et concentrée. Le brut est chromatographié sur gel de silice, éluant dichlorométhane acétate d'éthyle de 70/30 à 40/60. On obtient 2-tert-butyl-6-chloro-7-(N-pyridin-4-yl)sulfoximin-benzooxazole (0.36 g; 28%).
MS(ES+) *m*/*z* 364 (MH+).

### EXEMPLE 4

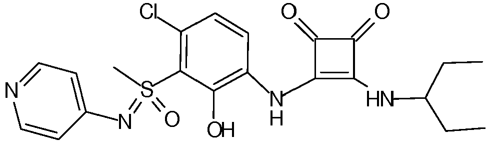

### 3-{4-Chloro-2-hydroxy-3-methane-[(N-pyridin-4-yl)-sulfoximin]-phenylamino}-4-(1-Ethyl-propylamino)-cyclobut-3-ene-1,2-dione

Une suspension de 3-(4-chloro-2-hydroxy-3-methane-[(N-pyridin-4-yl)-sulfoximin]-phenylamino)-4-ethoxy-cyclobut-3-ene-1,2-dione (114 mg; 0.27 mmol) et de 1-ethyl-propylamine (0.04 ml; 0.32 mmol) dans du méthanol (3 ml) est chauffée à 60°C pendant 24 heures. Le milieu réactionnel refroidi. Le précipité qui se forme est filtré et séché. Le composé obtenu est chromatographié sur gel de silice, éluant dichlorométhane méthanol 95/05. On obtient 3-{4-chloro-2-hydroxy-3-methane-[(N-pyridin-4-yl)-sulfoximin]-phenylamino}-4-(1-ethyl-propylamino)-cyclobut-3-ene-1,2-dione (85 mg; 67%).
1H NMR (400 MHz, DMSO): δ (ppm) 0.84-0.89 (m, 6H); 1.38-1.47 (m, 2H); 1.54-1.61 (m, 2H); 3.80 (s, 3H); 3.86 (t, J = 7.3 Hz, 1H); 6.20 (s, 1H); 6.99 (d, J = 6.8 Hz, 2H); 7.81 (d, J = 8.2 Hz, 1H); 8.24 (d, J = 6.8 Hz, 2H); 8.37 (d, J = 9.1 Hz, 1H); 9.33 (s, 1H). MS(ES+) *m*/*z* 463 (MH+)

### EXEMPLE 5

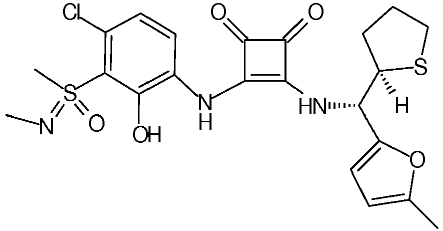

### 3-{4-Chloro-2-hydroxy-3-methane-[(N-methyl)-sulfoximin]-phenylamino}-4-{[(S)-(5-methyl-furan-2-yl)-(R)-tetrahydro-thiophen-2-yl-methyl]-amino}-cyclobut-3-ene-1,2-dione

A un mélange de 3-(4-chloro-2-hydroxy-3-methane-[(N-methyl)-sulfoximin]-phenylamino)-4-ethoxy-cyclobut-3-ene-1,2-dione (0.16 g; 0.45 mmol) dans du méthanol (3 ml) est rajoutée une solution de C-[(S)-C-(5-methyl-furan-2-yl)-C-(R)-tetrahydro-thiophen-2-yl]-methylamine (0.12 g; 0.61 mmol ; préparé selon le mode d'opératoire décrite dans WO 2013061004) dans du méthanol (1 ml). Au bout de 26 heures, de l'acétate d'éthyle est rajouté et le mélange est lavé deux fois avec une solution aqueuse à 1 M de dihydrogénophosphate de sodium. La phase organique est séchée sur sulfate de sodium anhydre, filtrée et concentrée. Le solide obtenu est chromatographié sur gel de silice (éluant heptane/acétone, de 10% à 50% d'acétone). Le solide obtenu est mis à sécher sous vide à l'étuve à 50°C. 3-{4-Chloro-2-hydroxy-3-methane-[(N-methyl)-sulfoximin]-phenylamino}-4-{[(S)-(5-methyl-furan-2-yl)-(R)-tetrahydro-thiophen-2-yl-methyl]-amino}-cyclobut-3-ene-1,2-dione (0.14 g; 61%) est obtenu de point de fusion 155°C. MS(ES+) *m*/*z* 510 (MH+).
1H NMR (400 MHz, DMSO): δ 1.81-1.85 (m, 1H); 1.91-1.94 (m, 1H); 2.01-2.06 (m, 2H); 2.27 (s, 3H); 2.79-2.81 (m, 2H); 2.90 (s, 3H); 3.70 (s, 3H); 3.87 (dt, J = 9.6, 6.1 Hz, 1H); 5.20 (t, J = 9.6 Hz, 1H); 6.07 (d, J = 3.0 Hz, 1H); 6.30 (d, J = 3.1 Hz, 1H); 6.98 (d, J = 8.7 Hz, 1H); 8.03 (dd, J = 8.7, 3.3 Hz, 1H); 8.89 (dd, J = 9.6, 4.6 Hz, 1H); 9.41 (d, J = 4.0 Hz, 1H).

### EXEMPLE 6

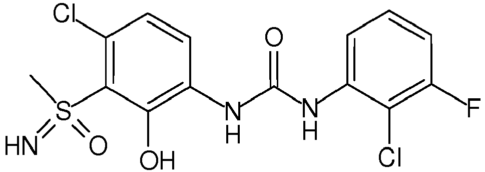

### 1-(2-chloro-3-fluoro-phenyl)-3-(4-chloro-2-hydroxy-3-methanesulfoximine-phenyl)-urea

A une solution de 6-amino-3-chloro-2-methanesulfoximin-phenol (0.19 g; 0.81 mmol) dans de l'acétonitrile (1.50 ml) et du N,N-diméthylformamide (0.50 ml), sous azote, est rajoutée une solution de 2-chloro-1-fluoro-3-isocyanato-benzene (0.15 g; 0.89 mmol) dans de l'acétonitrile (1 ml). Au bout de deux heures, le milieu réactionnel est concentré. Le résidu est purifié par HPLC/MS préparative (colonne C18 de 21mm de diamètre XSelect, eau/acétonitrile, 45% d'acétonitrile puis 60%) 1-(2-chloro-3-fluoro-phenyl)-3-(4-chloro-2-hydroxy-3-methanesulfoximine-phenyl)-urea (0.08 g; 25.2 %) est obtenu de point de fusion 203°C. MS(ES-) *m*/*z* 390 (MH-).
1H NMR (400 MHz, DMSO): δ 3.63 (s, 3H); 7.00 (d, J = 8.8 Hz, 1H); 7.08 (t, J = 8.7 Hz, 1H); 7.34 (q, J = 7.6 Hz, 1H); 7.98 (d, J = 8.5 Hz, 1H); 8.28 (d, J = 8.8 Hz, 1H); 9.27 (s, 1H); 9.30 (s, 1H); 11.64 (br s, 2H).

### EXEMPLE 7

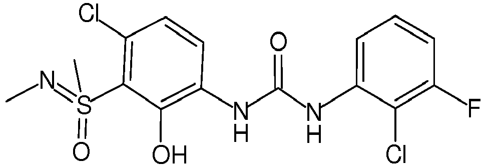

### 1-(2-Chloro-3-fluoro-phenyl)-3-{4-chloro-2-hydroxy-3-methanes-[(N-methyl)-sulfoximin]-phenyl}-urea

A une solution de 6-amino-3-chloro-2-methane-[(N-methyl)-sulfoximin]-phenol (0.16 g; 0.52 mmol) dans de l'acétonitrile (1.5 ml) et du N,N-diméthylformamide (0.50 ml) est rajoutée une solution de 2-chloro-1-fluoro-3-isocyanato-benzene (0.12 g; 0.68 mmol) dans de l'acétonitrile (1.5 ml). Au bout de 3 heures, le milieu réactionnel est concentré. Le résidu obtenu est chromatographiée sur gel de silice (éluant heptane/acétate d'éthyle, de 10% à 60% d'acétate d'éthyle). 1-(2-Chloro-3-fluoro-phenyl)-3-{4-chloro-2-hydroxy-3-methanes-[(N-methyl)-sulfoximin]-phenyl}-urea (0.10 g; 45%) est obtenu de point de fusion 107°C. MS(ES+) *m*/*z* 406 (MH+).
1H NMR (400 MHz, DMSO): δ 2.92 (s, 3H); 3.68 (s, 3H); 6.99 (d, J = 8.8 Hz, 1H); 7.08 (t, J = 8.7 Hz, 1H); 7.33-7.34 (m, 1H); 7.97 (d, J = 8.5 Hz, 1H); 8.28 (d, J = 8.8 Hz, 1H); 9.27 (s, 1H); 9.30 (s, 1H); 16.33 (br s, 1H).

### EXEMPLE 8

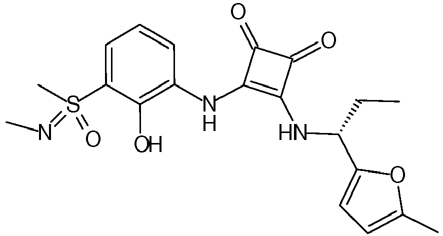

### 3-{2-hydroxy-3-methane-[(N-methyl)-sulfoximin]-phenylamino}-4-[(R)-1-(5-methyl-furan-2-yl)-propylamino]-cyclobut-3-ene-1,2-dione

A une suspension de 3-[3-(N,S-dimethylsulfonimidoyl)-2-hydroxy-anilino]-4-ethoxy-cyclobut-3-ene-1,2-dione (0.10 g; 0.31 mmol) dans du méthanol (3 ml) est ajouté à température ambiante de chlorhydrate de (R)-1-(5-methyl-furan-2-yl)-propylane (64.99 mg; 0.37 mmol) et de la triethylamine (51 µl; 0.37 mmol). Le milieu réactionnel est chauffé à 60°C pendant 1 heure 30. Le milieu réactionnel est concentré, le brut est chromatographiée sur gel de silice (éluant 10-60% méthanol dans dichlorométhane). 3-{2-Hydroxy-3-methane-[(N-methyl)-sulfoximin]-phenylamino]-4-[(R)-1-(5-methyl-furan-2-yl)-propylamino]-cyclobut-3-ene-1,2-dione (0.10 g; 77) est obtenu. MS(ES+) m/z 418 (MH+)
¹H NMR (DMSO-*d*₆) δ: 9.36 (s, 1H), 8.72 (dd, J = 9.0, 2.2 Hz, 1H), 8.04 (dt, J = 8.0, 1.7 Hz, 1H), 7.40 (dd, J = 8.2, 1.5 Hz, 1H), 6.90 (t, J = 8.0 Hz, 1H), 6.26 (t, J = 2.2 Hz, 1H), 6.06 (d, J = 3.6 Hz, 1H), 5.14 (q, J = 7.9 Hz, 1H), 3.46 (s, 3H), 2.83 (s, 3H), 2.27 (s, 3H), 2.02 - 1.81 (m, 2H), 0.93 (t, J = 7.3 Hz, 3H)

### 3-[3-(N,S-dimethylsulfonimidoyl)-2-hydroxy-anilino]-4-ethoxy-cyclobut-3-ene-1,2-dione

A une solution de 2-amino-6-(N,S-dimethylsulfonimidoyl)phenol (0.53 g; 2.65 mmol) dans ethanol (10 ml) est ajouté à température ambiante 3,4-diethoxy-3-cyclobutene-1,2-dione (0.59 ml; 3.97 mmol). Le milieu réactionnel est chauffé à 60°C pendant 24h. Le milieu réactionnel est filtré, 0.39g de produit attendu sous forme d'un solide jaune est récupérée. 3-[3-(N,S-dimethylsulfonimidoyl)-2-hydroxy-anilino]-4-ethoxy-cyclobut-3-ene-1,2-dione (0.39 g; 45%) est obtenu. MS(ES+) m/z 325 (MH+)

### 2-amino-6-(N,S-dimethylsulfonimidoyl)phenol

A une solution de 2-tert-butyl-7-methane-N-methyl-sulfoximin-benzooxazole (0.57 g; 2.14 mmol) dans 1,4-dioxanne (8.6 ml) et eau (2.28 ml) est ajouté à température ambiante l'acide sulfurique (2.11 ml; 39.42 mmol). Le milieu réactionnel est agité à 100°C pendant 4h puis 16h à 80°C. Le pH du milieu est ramené à pH=7 avec de la soude 10N, puis il est extré avec 50ml d'acétate d'éthyle. La phase organique est récupérée puis séché avec sulfate de magnésium, filtré et évaporé. 2-Amino-6-(N,S-dimethylsulfonimidoyl)phenol (0.54 g) est obtenue. MS(ES+) m/z 201 (MH+).

### 2-tert-butyl-7-methane-N-methyl-sulfoximin-benzooxazole

A une solution de 2-tert-butyl-6-chloro-benzooxazole-7-sulfoximine (550 mg; 2.18 mmol) dans N,N-diméthylformamide (11 ml) est ajoutée par petite portion hydride de sodium 60% dans l'huile (105 mg; 2.62 mmol). Le milieu réactionnel est agité 20 minutes à température ambiante puis iodométhane (258 µl; 4.14 mmol) est rajouté. Le milieu réactionnel est ensuite remis à température ambiante et agité pendant 20 minutes. Le milieu réactionnel est partitionné entre 50 ml d'eau et 50 ml d'acétate d'éthyle. La phase organique est lavée deux fois avec 20 ml de bicarbonate de sodium saturé et séchée sur sulfate de magnésium, filtrée et évaporée. 2-tert-Butyl-7-methane-N-methyl-sulfoximin-benzooxazole (570 mg; 98%) est obtenu. MS(ES+) m/z 267 (MH+).

### 2-tert-Butyl-7-methanesulfoximin-benzooxazole

A une solution dégazée 3 fois de 2-tert-butyl-6-chloro-benzooxazole-7-sulfoximine (1.00 g; 3.49 mmol) dans du tetrahydrofuranne (18 ml) est ajoutée de l'acétate de palladium(II) (39.14 mg; 0.17 mmol), fluoride de potassium (405.16 mg; 6.97 mmol) dilué dans EAU (7 ml) dégazé et 1,1,1,3,5,5,5-heptamethyltrisiloxane (3.10 g; 13.95 mmol) goutte à goutte. Le milieu est agité à température ambiante pendant 1 heure. Le milieu est purgé en azote puis de l'acétate de palladium (39.14 mg; 0.17 mmol) et 1,1,1,3,5,5,5-heptamethyltrisiloxane (3.10 g; 13.95 mmol) sont ajoutés puis le milieu est agité 16h à température ambiante. Le milieu est purgé à nouveau avec azote puis de l'acétate de palladium (39.14 mg; 0.17 mmol), 1,1,1,3,5,5,5-heptamethyltrisiloxane (3.10 g; 13.95 mmol), fluoride de potassium (405.16 mg; 6.97 mmol) sont ajoutés et le milieu est agité 30 minutes à température ambiante. Le milieu est transféré sur 30 ml de NaOH 3N à 0°C puis agité 4 heures à température ambiante. Le milieu est extraites 3 fois avec 50 ml d'éther diéthylique, les phases organiques sont rassemblées puis séchées sur sulfate de magnésium, filtrées et évaporées. Le brut est chromatographié sur colonne de silice (éluant avec le mélange de 0% à 70% Acétate d'éthyle dans heptane). 2-tert-Butyl-7-methanesulfoximin-benzooxazole (0.57 g; 64%) est obtenu.
MS(ES+) m/z 254 (MH+)

### EXEMPLE 9

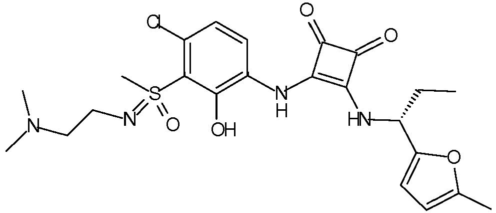

### 3-[4-chloro-3-[N-(2-dimethylaminoethyl)-S-methyl-sulfonimidoyl]-2-hydroxy-anilino]-4-ethoxy-cyclobut-3-ene-1,2-dione

A une suspension de 3-[4-chloro-3-[N-(2-dimethylaminoethyl)-S-methyl-sulfonimidoyl]-2-hydroxy-anilino]-4-ethoxy-cyclobut-3-ene-1,2-dione (180 mg; 0.43 mmol) dans méthanol (7.20 ml) à température ambiante est ajoutée de chlorhydrate de (R)-1-(5-methyl-furan-2-yl)-propylane (91.23 mg; 0.52 mmol) triethylamine (0.09 ml; 0.65 mmol). Le milieu réactionnel est chauffé à 60°C pendant deux heures. Le milieu réactionnel est concentré, le brut est chromatographiée sur gel de silice (5-10% méthanol dans dichlorométhane). 3-[4-Chloro-3-[N-(2-dimethylaminoethyl)-S-methyl-sulfonimidoyl]-2-hydroxy-anilino]-4-ethoxy-cyclobut-3-ene-1,2-dione (150 mg; 64%) est obtenu. MS(ES+) m/z 510 (MH+)
1H NMR δ (ppm)(DMSO-d6): 0.94-0.91 (3 H, m), 1.85 (1 H, dt, J = 14.12, 7.12 Hz), 1.94 (1 H, dd, J = 13.35, 7.62 Hz), 2.26 (3 H, d, J = 4.16 Hz), 2.56 (3 H, s), 2.85 (2 H, br s), 2.90 (1 H, s), 3.01 (1 H, d, J = 12.42 Hz), 3.22 (1 H, d, J = 14.18 Hz), 3.36 (3 H, d, J = 12.13 Hz), 5.16 (1 H, d, J = 8.68 Hz), 6.05 (1 H, s), 6.24 (1 H, d, J = 10.88 Hz), 6.47 (1 H, t, J = 7.18 Hz), 7.99 (1 H, dd, J = 14.72, 8.42 Hz), 8.90 (1 H, dd, J = 30.8, 8.83 Hz), 9,40 (1H, s).

### 3-[4-chloro-3-[N-(2-dimethylaminoethyl)-S-methyl-sulfonimidoyl]-2-hydroxy-anilino]-4-ethoxy-cyclobut-3-ene-1,2-dione

A une solution de 6-amino-3-chloro-2-[N-(2-dimethylaminoethyl)-S-methyl-sulfonimidoyl]phenol (190 mg; 0.65 mmol) dans ethanol (5.70 ml) est ajoutée à température ambiante 3,4-diethoxy-3-cyclobutene-1,2-dione (145 µl; 0.98 mmol). Le milieu réactionnel est chauffé à 60°C pendant 2 heures. L'éthanol est évaporé et le résidu est chromatographiée sur gel de silice (5-10% méthanol dans dichlorométhane). 3-[4-Chloro-3-[N-(2-dimethylaminoethyl)-S-methyl-sulfonimidoyl]-2-hydroxy-anilino]-4-ethoxy-cyclobut-3-ene-1,2-dione (130 mg; 48%) est obtenu. MS(ES+) m/z 418 (MH+)

### 6-Amino-3-chloro-2-[N-(2-dimethylaminoethyl)-S-methyl-sulfonimidoyl]phenol

A une solution de 2-[[(2-tert-Butyl-6-chloro-1,3-benzoxazol-7-yl)-methyl-oxo-sulfanylidene]amino]-N,N-dimethyl-ethanamine (760 mg; 2.12 mmol) dans 1,4-dioxanne (11.40 ml) et eau (3 ml) à température ambiante est ajoutée, gout à gout, de l'acide sulfurique concentré (2.1 ml; 39.11 mmol; 18.42 eq.). Le milieu réactionnel est agité à 60°C pendant 48 heures. Le milieu réactionnel est ramené à pH=8 avec de la soude 10M. Le produit est extrait 3 fois avec 25ml d'acétate d'éthyle. La phase organique est séchée sur sulfate de magnésium puis les solvants sont évaporés. Le résidu obtenu est chromatographié sur colonne de silice (2-10% méthanol dans dichlorométhane). 6-Amino-3-chloro-2-[N-(2-dimethylaminoethyl)-S-methyl-sulfonimidoyl]phénol (280 mg; 45%) est obtenu.
MS(ES+) m/z 292 (MH+)

### 2-[[(2-tert-Butyl-6-chloro-1,3-benzoxazol-7-yl)-methyl-oxo-sulfanylidene]amino]-N,N-dimethyl-ethanamine

A une suspension de 2-tert-butyl-6-chloro-benzooxazole-7-sulfoximine (2.00 g; 6.97 mmol) dans toluene (10 ml) est ajoutée à température ambiante (triphenylphosphanylidene)-acetonitrile (6.30 g; 20.92 mmol) et 2-dimethylaminoethanol (2.10 ml; 20.92 mmol). Le milieu est chauffé 24 heures à 120°C. Le milieu réactionnel est partitionné entre 25 ml d'eau et 25 ml d'acétate d'éthyle. La phase aqueuse est reextraite encore une fois avec 25 ml d'acétate d'éthyle. Les phases organiques sont rassemblées puis séchées sur le sulfate de magnésium, filtrées et évaporées. Le brut est chromatographié sur silice (2-10% méthanol dans dichlorométhane). 2-[[(2-tert-Butyl-6-chloro-1,3-benzoxazol-7-yl)-methyl-oxo-sulfanylidene]amino]-N,N-dimethyl-ethanamine (1.00 g; 40%) est obtenu. MS(ES+) m/z 359 (MH+).

### EXEMPLE 10

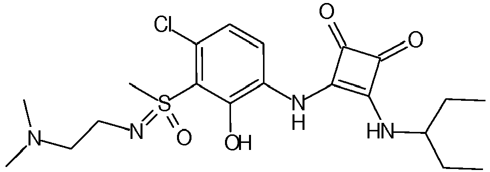

### 3-[4-chloro-3-[N-(2-dimethylaminoethyl)-S-methyl-sulfonimidoyl]-2-hydroxy-anilino]-4-(1-ethylpropylamino)cyclobut-3-ene-1,2-dione

A une suspension de 3-[4-chloro-3-[N-(2-dimethylaminoethyl)-S-methyl-sulfonimidoyl]-2-hydroxy-anilino]-4-ethoxy-cyclobut-3-ene-1,2-dione (80 mg; 0.19 mmol) dans méthanol (3.2 ml) est ajoutée à température ambiante 1-ethyl-propylamine (27 µl; 0.23 mmol). Le milieu réactionnel est chauffé à 60°C pendant 48 heures. Le milieu réactionnel est concentré, 100mg de brut sont purifiés par HPLC préparative. On obtient 3-[4-chloro-3-[N-(2-dimethylamino-ethyl)-S-methyl-sulfonimidoyl]-2-hydroxy-anilino]-4-(1-ethylpropylamino)cyclobut-3-ene-1,2-dione (25 mg; 28%).
1H NMR δ (ppm)(DMSO-d6): 0.93-0.86 (6 H, m), 1.52-1.42 (2 H, m), 1.67-1.57 (2 H, m), 2.55 (6 H, s), 2.93-2.81 (2 H, m), 3.04-2.97 (1 H, m), 3.22 (1 H, dt, J = 13.96, 4.56 Hz), 3.37 (3 H, s), 3.92-3.87 (1 H, m), 6.47 (1 H, d, J = 8.43 Hz), 8.03 (1 H, d, J = 8.43 Hz), 8.43 (1 H, d, J = 9.05 Hz), 9.33 (1 H, s). MS(ES+) m/z 457 (MH+)

### EXEMPLE 11

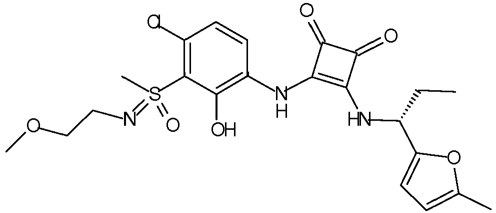

### 3-[4-chloro-2-hydroxy-3-[N-(2-methoxyethyl)-S-methyl-sulfonimidoyl]anilino]-4-[[(1R)-1-(5-methyl-2-furyl)propyl]amino]cyclobut-3-ene-1,2-dione

A une suspension de 3-[4-chloro-2-hydroxy-3-[N-(2-methoxyethyl)-S-methyl-sulfonimidoyl]anilino]-4-ethoxy-cyclobut-3-ene-1,2-dione (127 mg; 0.32 mmol) dans méthanol (5 ml) est ajoutée à température ambiante de chlorhydrate de (R)-1-(5-methyl-furan-2-yl)-propylane (66 mg; 0.38 mmol) et triethylamine (0.07 ml; 0.47 mmol). Le milieu réactionnel est chauffé à 50°C pendant 2 heures, puis il est concentré et le brut est chromatographiée sur gel de silice (l'éluant 30-60% acétate d'éthyle dans heptane). 3-[4-Chloro-2-hydroxy-3-[N-(2-methoxyethyl)-S-methyl-sulfonimidoyl]-anilino]-4-[[(1R)-1-(5-methyl-2-furyl)propyl]-amino]cyclobut-3-ene-1,2-dione (85 mg; 54%) est obtenu après le traitement avec l'éther-diisopropilique. MS(ES+) m/z 496 (MH+).
1H NMR δ (ppm)(DMSO-d6): 0.92 (3 H, t, J = 7.29 Hz), 1.89-1.84 (1 H, m), 1.95 (1 H, dd, J = 13.89, 7.02 Hz), 2.27 (3 H, s), 3.31 (3 H, m), 3.46-3.39 (2 H, m), 3.52 (2 H, s), 3.68 (2 H, s), 5.14 (1 H, q, J = 7.65 Hz), 6.06 (1 H, s), 6.26 (1 H, s), 7.01 (1 H, s), 8.03 (1 H, d, J = 8.72 Hz), 8.78 (1 H, d, J = 8.82 Hz), 9.36 (1 H, s).

### 3-[4-chloro-2-hydroxy-3-[N-(2-methoxyethyl)-S-methyl-sulfonimidoyl]anilino]-4-ethoxy-cyclobut-3-ene-1,2-dione

A une solution de 6-amino-3-chloro-2-methane-[(N-2-methoxy-ethylamino)-sulfoximin]-phenol (0.50 g; 1.79 mmol) dans ethanol (9.5 ml) est ajoutée à température ambiante 3,4-diethoxy-3-cyclobutene-1,2-dione (0.40 ml; 2.69 mmol). Le milieu réactionnel est chauffé à 50°C pendant 4 jours. Le milieu est concentré et le résidu est chromatographiée sur gel de silice (l'éluant 2-5% méthanol dans dichlorométhane). 3-[4-Chloro-2-hydroxy-3-[N-(2-methoxyethyl)-S-methyl-sulfonimidoyl]anilino]-4-ethoxy-cyclobut-3-ene-1,2-dione (127 mg; 17%) est obtenu. MS(ES+) m/z 403 (MH+).

### 6-Amino-3-chloro-2-methane-[(N-2-methoxy-ethylamino)-sulfoximin]-phenol

A une solution de 2-tert-butyl-6-chloro-7-methane-N-(2-methoxy-ethylamino)-sulfoximin-benzo-oxazole (0.70 g; 2.03 mmol) dans 1,4-dioxanne (4.9 ml) et eau (1.12 ml) est ajoutée goutte à goutte à température ambiante de l'acide sulfurique concentré (0.84 ml; 15.72 mmol). Le milieu réactionnel est chauffé à 110°C pendant 4 heures. Le milieu réactionnel est concentré et 28 ml de soude 1N sont ajoutés (pH à 7), puis extrait avec 50ml d'acétate d'éthyle. La phase organique est séchée sur sulfate de magnésium, filtrée et les solvants sont évaporés. 6-Amino-3-chloro-2-methane-[(N-2-methoxy-ethylamino)-sulfoximin]-phenol (0.40 g; 70%) est obtenu. MS(ES+) m/z 279 (MH+).

### 2-tert-butyl-6-chloro-7-methane-N-(2-methoxy-ethylamino)-sulfoximin-benzooxazole

A une solution de 2-tert-butyl-6-chloro-benzooxazole-7-sulfoximine (1.00 g; 3.49 mmol) dans N,N-dimethylformamide (15 ml) est ajoutée à température ambiante d'hydride de sodium 60% dans l'huile (0.21 g; 5.23 mmol). Le milieu est agité 10 minutes à température ambiante puis 2-bromoethyl méthyl éther (0.49 ml; 5.23 mmol) est ajouté. Le milieu réactionnel est agité à température ambiante pendant 24 heures. Hydride d'sodium 60% dans l'huile (21 mg; 0.52 mmol) et 2-bromoethyl méthyl éther (0.07 ml; 0.70 mmol) supplémentaires sont ajoutés au milieu réactionnel et le milieu est agité 2h à 60°C. Le milieu réactionnel est partitionnée entre 30 ml d'eau et 30ml d'acétate d'éthyle, la phase organique est lavée 3 fois avec 20ml d'eau puis séchée sur sulfate de magnésium, filtrée et évaporée. Le résidu est chromatographié sur gel de silice (l'éluant 30-50% acétate d'éthyle dans heptane). 2-tert-Butyl-6-chloro-7-methane-N-(2-methoxy-ethylamino)-sulfoximin-benzooxazole (0.70 g; 58%) est obtenu. MS(ES+) m/z 345 (MH+).

### EXEMPLE 12

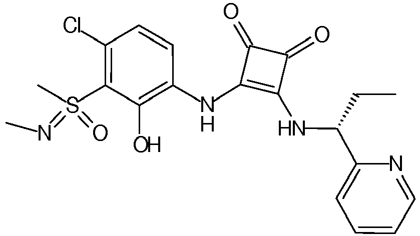

### (-)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione

Un mélange de (-)-3-(4-chloro-2-hydroxy-3-methane-[(N-methyl)-sulfoximin]-phenylamino)-4-ethoxy-cyclobut-3-ene-1,2-dione (216 mg; 0.60 mmol ; préparé à partie de énantiomère A, (-)-2-tert-butyl-6-chloro-7-methanesulfoximin-benzooxazole) et de (R)-1-pyridin-2-yl-propylamine chlorhydrate (125 mg; 0.72 mmol) dans méthanol (5 ml) et en présence de triethylamine (100 µl; 0.72 mmol) est chauffé à 50°C pendant 7 heures et agité à température ambiante pendant 16h. Le solvant est évaporé et le résidu est chromatographié sur gel de silice HP (colonne puriFlash PF-15HP/25g, CombiFlash) élué au dichlorométhane / acétate d'éthyle (90/10 à 70/30). Le solide est repris avec un peu d'acétate d'éthyle, filtré et séché sous vide à 45°C. (-)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione (156 mg; 57%) est obtenu. MS(ES+) *m*/*z* 449 (MH+); t=5.37 min; 100% ; [α]_{D} = -32.3 ° (c=10 mg/ml; ethanol).
1H NMR (400 MHz, DMSO): δ (ppm) 0.85-0.89 (t, J = 7.3 Hz, 3H), 1.84-1.97 (m, 2H), 2.91 (s, 3H), 3.69 (s, 3H), 5.23-5.29 (q, J = 7.4 Hz, 1H), 6.98 (d, J = 8.7 Hz, 1H), 7.34-7.37 (m, 1H), 7.41 (d, J = 7.8 Hz, 1H), 7.81-7.85 (td, J = 7.7-1.6 Hz, 1H), 8.01 (d, J = 8.7 Hz, 1H), 8.63 (d, J = 4.4 Hz, 1H), 9.05-9.08 (d, J = 9.1 Hz, 1H)

### EXEMPLE 13

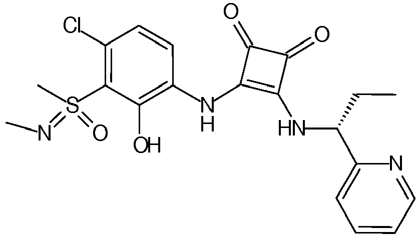

### (+)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione

Un mélange de (+)-3-(4-chloro-2-hydroxy-3-methane-[(N-methyl)-sulfoximin]-phenylamino)-4-ethoxy-cyclobut-3-ene-1,2-dione (76 mg; 0.21 mmol ; préparé à partie de énantiomère B, (+)-2-tert-butyl-6-chloro-7-methanesulfoximin-benzooxazole) et de (R)-1-pyridin-2-yl-propylamine chlorhydrate (44 mg; 0.25 mmol) dans méthanol (1.50 ml) et en présence de triethylamine (0.04 ml; 0.25 mmol) est chauffé à 50°C pendant 7 heures et agité à température ambiante pendant 16h. Le solvant est évaporé et le résidu est chromatographié sur gel de silice HP (colonne puriFlash PF-15HP/4G, CombiFlash) élué au dichlorométhane / acétate d'éthyle (90/10 à 70/30). Le solide est repris avec un peu d'heptane / acétate d'éthyle, filtré et séché sous vide à 45°C. (+)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione (41 mg; 42%) est obtenu; MS(ES+) *m*/*z* 449 (MH+); t=5.34 min, 98.6%.
1H NMR (400 MHz, DMSO): δ (ppm) 0.85-0.89 (t, J = 7.3 Hz, 3H), 1.84-1.99 (m, 2H), 2.91 (s, 3H), 3.69 (s, 3H), 5.23-5.29 (q, J = 7.4 Hz, 1H), 6.98 (d, J = 8.7 Hz, 1H), 7.34-7.37 (m, 1H), 7.41 (d, J = 7.8 Hz, 1H), 7.81-7.85 (td, J = 7.7-1.6 Hz, 1H), 8.01 (d, J = 8.7 Hz, 1H), 8.63 (d, J = 4.4 Hz, 1H), 9.05-9.08 (d, J = 9.1 Hz, 1H).

### EXEMPLE 14

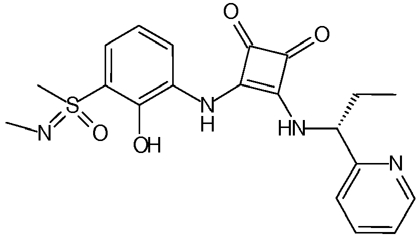

### (-)-3-{2-Hydroxy-3-methane-[(N-methyl)-sulfoximin]-phenylamino}-4-[(R)-1-(pyridin-2-yl)-propylamino[-cyclobut-3-ene-1,2-dione

Un mélange de (-)-3-(2-hydroxy-3-methane-[(N-methyl)-sulfoximin]-phenylamino)-4-ethoxy-cyclobut-3-ene-1,2-dione (586 mg; 1.81 mmol; préparé à partie de énantiomère A, (-)-2-tert-butyl-6-chloro-7-methanesulfoximin-benzooxazole) et de (R)-1-pyridin-2-yl-propylamine chlorhydrate (374 mg; 2.17 mmol) dans méthanol (10 ml) et en présence de triethylamine (300 µl; 2.17 mmol) est chauffé à 50°C pendant 2 jours. Le solvant est évaporé et le résidu est chromatographié sur gel de silice HP (colonne puriFlash PF-15HP/40G, CombiFlash) élué au dichlorométhane / méthanol (98/2 à 95/5). Le solide est repris avec un peu d'heptane / acétate d'éthyle, filtré et séché sous vide à 45°C. (-)-3-{2-Hydroxy-3-methane-[(N-methyl)-sulfoximin]-phenylamino]-4-[(R)-1-(pyridin-2-yl)-propylamino]-cyclobut-3-ene-1,2-dione (175 mg; 23%) est obtenu ; MS(ES+) *m*/*z* 415 (MH+) ; t=4.29 min; 98.51% ; [α]_{D} = -69.4 ° (c=8,5 mg/ml).
1H NMR (400 MHz, DMSO): δ (ppm) 0.86-0.89 (t, J = 7.3 Hz, 3H), 1.83-1.98 (m, 2H), 2.83 (s, 3H), 3.44 (s, 3H), 5.24-5.30 (q, J = 7.3 Hz, 1H), 6.88-6.92 (t, J = 8.1 Hz, 1H), 7.34-7.39 (m, 1H), 7.41-7.43 (t, J = 6.2 Hz, 1H), 7.81-7.86 (td, J = 7.7-1.6 Hz, 1H), 8.0-8.02 (d, J = 8.8 Hz, 1H), 8.63-8.64 (d, J = 4.2 Hz, 1H), 9.0-9.02 (d, J = 9.2 Hz, 1H).

### EXEMPLE 15

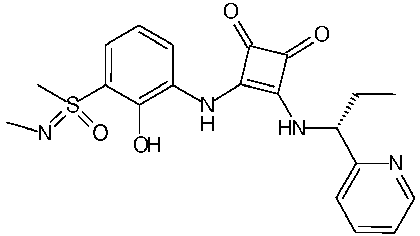

### (-)-3-{2-Hydroxy-3-methane-[(N-methyl)-sulfoximin]-phenylamino}-4-[(R)-1-(pyridin-2-yl)-propylamino]-cyclobut-3-ene-1,2-dione

Un mélange de (+)-3-(2-hydroxy-3-methane-[(N-methyl)-sulfoximin]-phenylamino)-4-ethoxy-cyclobut-3-ene-1,2-dione (150 mg; 0.46 mmol; préparé à partie de énantiomère B, (+)-2-tert-butyl-6-chloro-7-methanesulfoximin-benzooxazole) et de (R)-1-pyridin-2-yl-propylamine chlorhydrate (96 mg; 0.55 mmol) dans méthanol (4 ml) et en présence de triethylamine (77 µl; 0.55 mmol) est chauffé à 50°C pendant 14 heures puis à 55°C pendant 2 jours. Le solvant est évaporé et le résidu est chromatographié sur gel de silice avec dépôt solide (colonne puriFlash IR-50SI/12G, CombiFlash) élué au dichlorométhane / méthanol (98/2 à 95/5). Le solide est repris avec un peu d'heptane / acétate d'éthyle, filtré et séché sous vide à 45°C. (-)-3-{2-Hydroxy-3-methane-[(N-methyl)-sulfoximin]-phenylamino}-4-[(R)-1-(pyridin-2-yl)-propylamino]-cyclobut-3-ene-1,2-dione (158 mg; 81%) est obtenu ; MS(ES+) *m*/*z* 415 (MH+) ; t=0.92 min; [414] ; [α]_{D} = -5.2 ° (c=10,1 mg/ml).
1H NMR (400 MHz, DMSO): δ (ppm) 0.85-0.89 (t, J = 7.3 Hz, 3H), 1.83-1.99 (m, 2H), 2.83 (s, 3H), 3.45 (s, 3H), 5.24-5.30 (q, J = 7.3 Hz, 1H), 6.88-6.92 (t, J = 8.1 Hz, 1H), 7.34-7.39 (m, 1H), 7.41-7.43 (t, J = 6.2 Hz, 1H), 7.81-7.86 (td, J = 7.7-1.6 Hz, 1H), 8.0-8.02 (d, J = 8.8 Hz, 1H), 8.63-8.64 (d, J = 4.2 Hz, 1H), 9.0-9.02 (d, J = 9.2 Hz, 1H).

### EXEMPLE 16

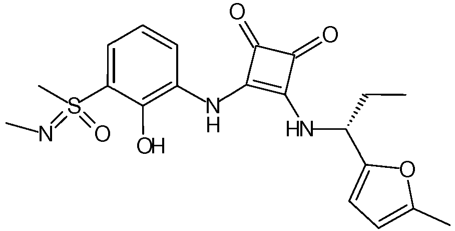

### (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione

Un mélange de (-)-3-(2-hydroxy-3-methane-[(N-methyl)-sulfoximin]-phenylamino)-4-ethoxy-cyclobut-3-ene-1,2-dione (586 mg; 1.81 mmol; préparé à partie de énantiomère A, (-)-2-tert-butyl-6-chloro-7-methanesulfoximin-benzooxazole) et de (R)-1-(5-methyl-furan-2-yl)-propylane chlorhydrate (381 mg; 2.17 mmol) dans méthanol (10 ml) et en présence de triethylamine (301 µl; 2.17 mmol) est chauffé à 50°C pendant 15 heures. Le solvant est évaporé et le résidu est chromatographié sur gel de silice HP (colonne puriFlash PF-15HP/40G, CombiFlash) élué au dichlorométhane / méthanol (98/2 à 95/5). Le solide est repris avec un peu d'heptane / acétate d'éthyle, filtré et séché sous vide à 45°C. (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione (751 mg; 98%) est obtenu; MS(ES+) *m*/*z* 418 (MH+); t=5.97 min; 99.19% ; [α]_{D} = +22.0 ° (c=10,2 mg/ml).
1H NMR (400 MHz, DMSO): δ (ppm) 0.90-0.94 (t, J = 7.3 Hz, 3H), 1.80-1.89 (m, 1H), 1.91-1.99 (m, 1H), 2.27 (s, 3H), 2.82 (s, 3H), 3.45 (s, 3H), 5.10-5.16 (q, J = 7.7 Hz, 1H), 6.05 (dd, J = 1.0 Hz, 1H), 6.26 (d, J = 3.0 Hz, 1H), 6.88-6.92 (t, J = 8.0 Hz, 1H), 7.39-7.41 (d, J = 8.1 Hz, 1H), 8.03-8.05 (d, J = 7.8 Hz, 1H), 8.70-8.72 (d, J = 9.0 Hz, 1H), 9.36 (s, 1H).
MS(ES+) *m*/*z* 418 (MH+)

### EXEMPLE 17

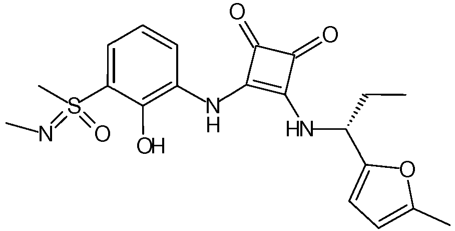

### (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione

Un mélange de (+)-3-(2-hydroxy-3-methane-[(N-methyl)-sulfoximin]-phenylamino)-4-ethoxy-cyclobut-3-ene-1,2-dione (150 mg; 0.46 mmol; préparé à partie de énantiomère B, (+)-2-tert-butyl-6-chloro-7-methanesulfoximin-benzooxazole) et de (R)-1-(5-methyl-furan-2-yl)-propylane chlorhydrate (97 mg; 0.55 mmol) dans méthanol (4 ml) et en présence de triethylamine (77 µl; 0.55 mmol) est chauffé à 50°C pendant 12 heures. Le solvant est évaporé et le résidu est chromatographié sur gel de silice avec dépôt solide (colonne puriFlash IR-50SI/12G, CombiFlash) élué au dichlorométhane / méthanol (98/2 à 95/5). Le solide est repris avec un peu d'heptane / acétate d'éthyle, filtré et séché sous vide à 45°C. (+)-3-{2-Hydroxy-3-methane-[(N-methyl)-sulfoximin]-phenylamino}-4-[(R)-1-(5-methyl-furan-2-yl)-propyl-amino]-cyclobut-3-ene-1,2-dione (158 mg; 81%) est obtenu ; MS(ES+) *m*/*z* 418 (MH+) ; t=5.98 min; 99.66% ; [α]_{D} = +94.1° (c=10,1 mg/ml)
1H NMR (400 MHz, DMSO): δ (ppm) 0.90-0.94 (t, J = 7.3 Hz, 3H), 1.80-1.89 (m, 1H), 1.91-1.99 (m, 1H), 2.27 (s, 3H), 2.82 (s, 3H), 3.45 (s, 3H), 5.10-5.16 (q, J = 7.7 Hz, 1H), 6.05 (dd, J = 1.0 Hz, 1H), 6.26 (d, J = 3.0 Hz, 1H), 6.88-6.92 (t, J = 8.0 Hz, 1H), 7.38-7.41 (dd, J = 8.2-1.4 Hz, 1H), 8.03-8.05 (d, J = 7.8 Hz, 1H), 8.70-8.73 (d, J = 9.0 Hz, 1H), 9.35 (s, 1H).

### EXEMPLE 18

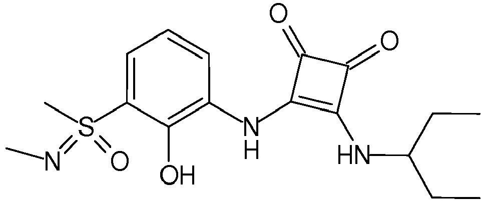

### (-)-3-(2-Hydroxy-3-methane-[(N-methyl)-sulfoxim]-phenylamino-[4-(1-ethyl-propylamino]-cyclobut-3-ene-1,2-dione

Un mélange de (-)-3-(2-hydroxy-3-methane-[(N-methyl)-sulfoximin]-phenylamino)-4-ethoxy-cyclobut-3-ene-1,2-dione (586 mg; 1.81 mmol), préparé à partir de énantiomère A, (-)-2-tert-butyl-6-chloro-7-methanesulfoximin-benzooxazole), et de 1-ethyl-propylamine (0.25 ml; 2.17 mmol) dans méthanol (10 ml) est chauffé à 50°C pendant 15 heures. Le solvant est évaporé et le résidu est chromatographié sur gel de silice HP (colonne puriFlash PF-15HP/40G, CombiFlash) élué au dichlorométhane / méthanol (98/2 à 95/5). Le solide est repris avec un peu d'heptane / acétate d'éthyle, filtré et séché sous vide à 45°C. (-)-3-(2-Hydroxy-3-methane-[(N-methyl)-sulfoxim]-phenylamino-[4-(1-ethyl-propylamino]-cyclobut-3-ene-1,2-dione (584 mg; 88%) est obtenu sous forme d'un solide jaune pâle ; MS(ES-) *m*/*z* 364 (MH-); t=5.20 min; 99.57% ; [α]_{D} = -45.8 ° (c=10,0 mg/ml).
1H NMR (400 MHz, DMSO): δ (ppm) 0.86-0.90 (t, J = 7.4 Hz, 3H), 0.88-0.92 (t, J = 7.3 Hz, 3H), 1.42-1.53 (m, 2H), 1.58-1.68 (m, 2H), 2.83 (s, 3H), 3.46 (s, 3H), 3.85-3.94 (m, 1H), 6.88-6.92 (t, J = 8.0 Hz, 1H), 7.37-7.40 (dd, J = 8.7 Hz, 1H), 8.07-8.09 (d, J = 8.1-1.0 Hz, 1H), 8.06-8.08 (d, J = 7.2 Hz, 1H), 8.23-8.25 (d, J = 9.0 Hz, 1H), 9.30 (s, 1H)

### EXEMPLE 19

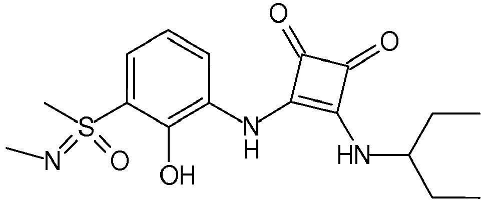

### (+)-3-(2-Hydroxy-3-methane-[(N-methyl)-sulfoxim]-phenylamino-[4-(1-ethyl-propylamino]-cyclobut-3-ene-1,2-dione

Un mélange de (+)-3-(2-hydroxy-3-methane-[(N-methyl)-sulfoximin]-phenylamino)-4-ethoxy-cyclobut-3-ene-1,2-dione (150 mg; 0.46 mmol; préparé à partir de énantiomère B, (+)-2-tert-butyl-6-chloro-7-methanesulfoximin-benzooxazole et de 1-ethyl-propylamine (65 µl; 0.55 mmol) dans méthanol (4 ml) est chauffé à 50°C pendant 12 heures. Le solvant est évaporé et le résidu est chromatographié sur gel de silice avec dépôt solide (colonne puriFlash IR-50SI/12G, CombiFlash) élué au dichlorométhane / méthanol (98/2 à 95/5). Le solide est repris avec un peu d'heptane / acétate d'éthyle, filtré et séché sous vide à 45°C. (+)-3-(2-Hydroxy-3-methane-[(N-methyl)-sulfoxim]-phenylamino-[4-(1-ethyl-propylamino]-cyclobut-3-ene-1,2-dione (156 mg; 91%) est obtenu ; MS(ES+) *m*/*z* 366 (MH+) ; t=5.22 min; 99.26% ; [α]_{D} = +40.8 ° (c=10,2 mg/ml).
1H NMR (400 MHz, DMSO): δ (ppm) 0.86-0.90 (t, J = 7.4 Hz, 3H), 0.88-0.92 (t, J = 7.3 Hz, 3H), 1.42-1.53 (m, 2H), 1.58-1.68 (m, 2H), 2.83 (s, 3H), 3.46 (s, 3H), 3.85-3.94 (m, 1H), 6.88-6.92 (t, J = 8.0 Hz, 1H), 7.37-7.40 (dd, J = 8.1-1.2 Hz, 1H), 8.07-8.09 (d, J = 8.1-1.0 Hz, 1H), 8.06-8.08 (d, J = 7.2 Hz, 1H), 8.23-8.25 (d, J = 9.0 Hz, 1H), 9.30 (s, 1H)

### EXEMPLE 20

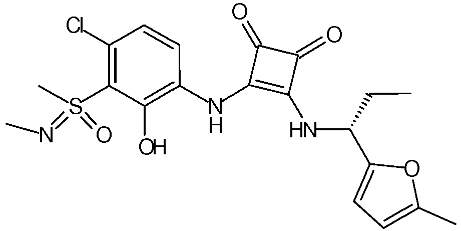

### (+)-3-{4-Chloro-2-hydroxy-3-methane-[(N-methyl)-sulfoximin]-phenylamino}-4-[(R)-1-(5-methyl-furan-2-yl)-propylamino[-cyclobut-3-ene-1,2-dione

Un mélange de (-)-3-(4-chloro-2-hydroxy-3-methane-[(N-methyl)-sulfoximin]-phenylamino)-4-ethoxy-cyclobut-3-ene-1,2-dione (270 mg; 0.75 mmol), préparé à partir de énantiomère A, (-)-2-tert-butyl-6-chloro-7-methanesulfoximin-benzooxazole), et de (R)-1-(5-methyl-furan-2-yl)-propylane chlorhydrate (158.6 mg; 0.90 mmol) dans méthanol (5 ml) et en présence de triethylamine (125 µl; 0.90 mmol) est chauffé à 50°C pendant 38 heures. Le solvant est évaporé et le résidu est chromatographié sur gel de silice HP (colonne puriFlash PF-15HP/25g, CombiFlash) élué au dichlorométhane / acétate d'éthyle (95/5 à 90/10). Le solide est repris avec un peu d'heptane / acétate d'éthyle, filtré et séché sous vide à 45°C. (-)-3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione (228 mg; 65%) est obtenu sous forme d'un solide jaune pâle ; t=17.1 min; 99.6% ; [α]_{D} = +30.8 ° (c=2,5 mg/ml).
1H NMR (400 MHz, DMSO): δ (ppm) 0.90-0.94 (t, J = 7.3 Hz, 3H), 1.82-1.99 (m, 2H), 2.27 (s, 3H), 2.90 (s, 3H), 3.69 (s, 3H), 5.10-5.16 (q, J = 7.7 Hz, 1H), 6.06 (dd, J = 1.0 Hz, 1H), 6.26 (d, J = 3.0 Hz, 1H), 6.97-6.99 (d, J = 8.7 Hz, 1H), 8.03-8.05 (d, J = 8.7 Hz, 1H), 8.75-8.77 (d, J = 9.0 Hz, 1H), 9.35 (s, 1H).

### TESTS BIOLOGIQUES

### EXEMPLE 21 : Affinité in vitro

L'affinité *In Vitro* des composés de la présente invention pour les récepteurs CXCR1 et CXCR2 est déterminée sur un test fonctionnel de type recrutement de la β-arrestine après activation du récepteur.

Il a été démontré que l'activation par CXCL8 du récepteur CXCR2 dans les cellules de la lignée PathHunter HEK293-CXCR2 ou du récepteur CXCR1 dans les cellules de la lignée U2OS h CXCR1 β-arrestine conduit au recrutement de la β-arrestine (Richardson, R. M., R. J. Marjoram, L. S. Barak, R. Snyderman. 2003. Role of the cytoplasmic tails of CXCR1 and CXCR2 in mediating leukocyte migration, activation, and regulation. J. Immunol. 170 : 2904.- 2911.)

Pour évaluer l'interaction directe du récepteur CXCR2 ou CXCR1 avec la β-arrestine 2, un test de recrutement β-arrestine 2 pour CXCR2 ou CXCR1 basé sur la complémentation de l'enzyme β-galactosidase (Olson KR, Eglen RM. Beta galactosidase complementation: a cell-based luminescent assay platform for drug discovery. Assay Drug Dev Technol. 2007 Feb; 5(1) ; 137-44), tel qu'établi par DiscoveRx Corporation a été utilisé. La stimulation de ces deux lignées cellulaires avec CXCL8 (10 nM) induit le recrutement de la β-arrestine 2, comme indiqué par une augmentation significative du facteur d'induction. Tous les antagonistes CXCR2 sont testés de manière dose-dépendante et la concentration correspondant à 50% d'inhibition de la réponse est déterminée (IC₅₀ = concentration de demi inhibition).

Test de Recrutement de la β-arrestine : les cellules « PathHunter HEK293-CXCR2 » ou « U2OS hCXCR1 β-arrestine » (DiscoveRx Corporation) ont été ensemencées une nuit à 10 000 cellules / puits (384 puits format) dans 20 µl de milieu Opti MEM I. Une préincubation avec l'antagoniste ou le véhicule de 30 min à 37 ° C et 5% de CO2 a été suivie par 60 minutes de stimulation par CXCL8 à 37 ° C et 5% de CO2. Les cellules ont ensuite été placées à température ambiante pendant 30 minutes. Le réactif de détection PathHunter (DiscoveRx Corporation) a été ajouté. Après une incubation de 60 min à température ambiante, la β-galactosidase induite par la luminescence lors de l'interaction β-arrestine-CXCR2 a été mesurée pour 0,3 s dans un Envision 2102 Multilabel Reader (PerkinElmer Life and Analytical Sciences). Les données sont analysées par une procédure de courbe non linéaire utilisant le logiciel d'exploitation XLFit4 (IDBS) et les IC50 sont déterminées.

**Tableau 3**

| **n° exemple** | **CXCR2 IC50 (nM)** | **CXCR1 IC50 (nM)** |
|---|---|---|
| 1 | A | C |
| 2 | A | C |
| 3 | B | D |
| 4 | B | D |
| 5 | B | C |
| 6 | B | D |
| 7 | B | D |
| 8 | B | C |
| 9 | B | D |
| 10 | B | D |
| 11 | B | D |
| 12 | A | C |
| 13 | B | D |
| 14 | B | C |
| 15 | B | D |
| 16 | A | C |
| 17 | B | D |
| 18 | B | D |
| 19 | B | D |
| 20 | A | C |

| | | |
|---|---|---|
| A : IC50 < 20nM ; B : IC50 > ou = 20nM C : IC50 < 200nM ; D : IC50 > ou = 200nM | | |

### EXEMPLE 22 : Migration des neutrophiles

### Purification et mise en culture des Neutrophiles Humains

Les préparations de sang périphérique (18-24 heures) sont collectées dans des cliniques dûment enregistrées ou des laboratoires cliniques auprès de volontaires sains qui ont consenti à la réutilisation de leur sang pour la recherche scientifique (Fournisseur : Biopredic International). Les neutrophiles sont séparés du sang total par une sélection positive (Whole Blood CD15 MicroBeads: MiltenyiBiotech, Référence : 130-091-058). Les cellules sont resuspendues à raison de 4x10⁶ cellules par mL dans le milieu de culture RPMI 1640 (Invitrogen) complémenté avec 5 % de sérum de veau foetal (SVF) filtré et inactivé par la chaleur (Invitrogen)

### Chemotaxis Assay

Les expériences ont été réalisées dans des plaques à filtres « HTS Transwell 96 Well Permeable Supports 3-µm pore size » (Corning, Référence : 3386). Les doses réponses d'Antagonistes CXCRS sont appliquées dans les compartiments inférieurs des plaques en présence de 10 nM d'IL-8 (1-72 aa, R&D Systems, Cat. N° IL-208). Puis les neutrophiles sont ensemencés dans le compartiment supérieur (2x10⁵ cellules/puits). Après 1h d'incubation à 37°C et 5% de CO2, le nombre de cellules viables ayant franchies la membrane et se retrouvant dans le compartiment inférieur est déterminé par une méthode colorimétrique (CellTiter 96® AQᵤₑₒᵤₛ One Solution Cell Proliferation Assay - PROMEGA Cat#G3581). Chaque condition de traitement est évaluée en double exemplaire. Les IC50 sont calculées avec le Logiciel XLFit4

**Tableau 4**

| **n° exemple** | **Migration des neutrophiles humains IC50 (nM)** |
|---|---|
| 1 | E |
| 2 | E |
| 3 | ND |
| 4 | ND |
| 5 | E |
| 6 | ND |
| 7 | ND |
| 8 | F |
| 9 | ND |
| 10 | ND |
| 11 | ND |
| 12 | E |
| 13 | ND |
| 14 | F |
| 15 | ND |
| 16 | F |
| 17 | ND |
| 18 | F |
| 19 | ND |
| 20 | E |

| | |
|---|---|
| ND : non determiné ; E : IC50<300nM ; F : IC50> ou =300nM | |

## Revendications

1. Composé répondant à la formule générale (I) suivante, ou un de ses sels ou un de ses énantiomères: 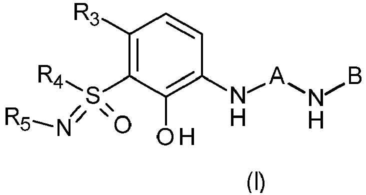 dans laquelle :
A represente 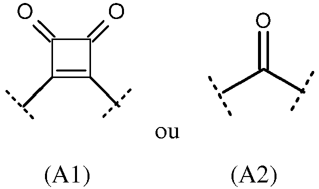
B represente 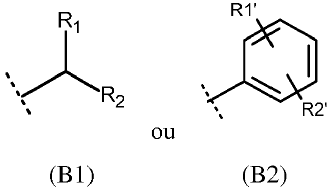
avec
R1' et R2', identiques ou différents, représentent un hydrogène, un halogène, un alkyle de C1 à C5 non substitué ou substitué par un ou plusieurs atomes de fluor, un alkoxy de C1 à C5, OCF3, OH, CN, NR11R12.
R1 et R2, identiques ou différents, représentent :
- un hydrogène,
- un alkyle de C1 à C5, non substitué ou substitué par un ou plusieurs groupes choisis parmi F, OH, OCH3 et NR11R12 ; R11 et R12 ayant la signification donnée ci-après, étant entendu que lorsque le radical alkyle de C1 à C5 n'est substitué que par un ou plusieurs atomes de fluor, il s'agit d'un radical alkyle fluoré ou d'un radical alkyle perfluoré de C1 à C5,
- un alkyle de C1 à C5 dans lequel un atome de carbone est remplacé par un atome d'oxygène ou par un atome de soufre, ledit alkyle de C1 à C5 étant non substitué ou substitué par un ou plusieurs groupes choisis parmi F, OH, NR11R12, R11 et R12 ayant la signification donnée ci-après,
- un cycloalkyle de C3 à C8
- un alcyne de C2 à C5, non substitué ou substitué par un ou plusieurs groupes choisis parmi F, OH, phényle, NR11R12, R11 et R12 ayant la signification donnée ci-après,
- un cycloalkyle répondant à l'une des formules (1), (2), (3), (4), (5) ou (6) suivantes dans lesquelles R5', X et X' ont les significations donnée ci-après : 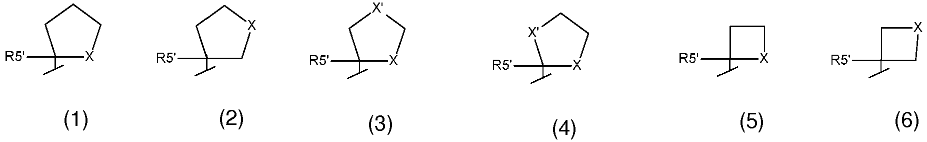
- un cycle aromatique ou heteroaromatique sélectionné dans le groupe constitué par les cycles répondant aux formules (a) à (o) suivantes dans lesquelles R7, R7a, Y et Z ont les significations données ci-après: 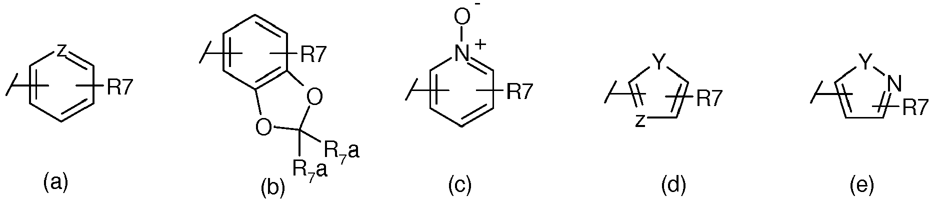 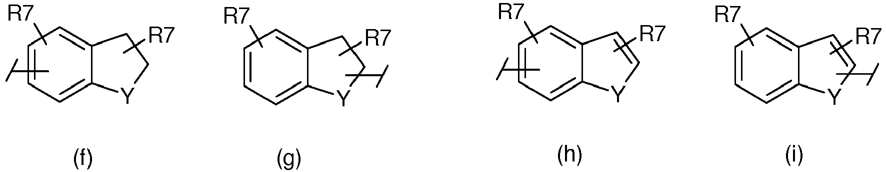 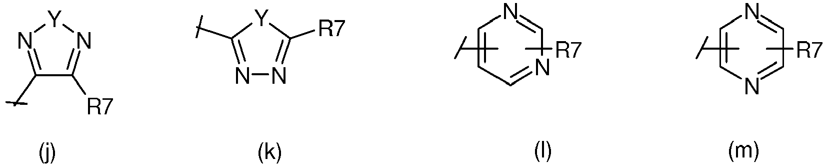 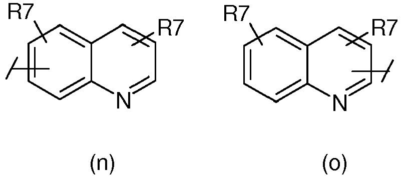
R7 peut être présent plusieurs fois sur un cycle, et au maximum, autant de fois qu'il y a d'atomes substituables. Les significations de chaque substituant R7 peuvent être identiques ou différentes.
R3 représente un hydrogène, un halogène, un alkyle de C1 à C5, un alkoxy de C1 à C5, - CF3, -OCF3, -OH, -NO2, -CN
R4 et R5, identiques ou différents, représentent :
- un hydrogène,
- un alkyle de C1 à C8, non substitué ou substitué par un ou plusieurs groupes choisis parmi F, OH, NR11R12, R11 et R12 ayant la signification donnée ci-après,
- un alkyle de C1 à C8 dans lequel un atome de carbone est remplacé par un atome d'azote, par un atome d'oxygène ou par un atome de soufre, ledit alkyle de C1 à C8 étant non substitué ou substitué par un ou plusieurs groupes choisis parmi F, OH, NR11R12, R11 et R12 ayant la signification donnée ci-après,
- un cycloalkyle de C3 à C8,
- un cycloalkyle de C3 à C8 dont l'un des atomes de carbones est remplacé par un atome d'oxygène ou par un atome d'azote substitué par un radical R7a,
- un hétérocycloalkyle de 5 à 7 atomes cycliques,
- un cycloalkylalkyle, le cycloalkyle étant de C3 à C8 et l'alkyle de C1 à C8,
- un phényle,
- un phényle substitué par un radical R7,
- un heteroaryle,
- un arylalkyle, l'alkyle étant de C1-C5,
- un heteroarylalkyle, l'alkyle étant de C1-C5,
ou encore
R4 et R5 représentent une chaine -(CH₂)ₘ- formant un cycle contenant de 5 à 8 atomes avec les atomes de soufre et d'azote auxquels ils sont respectivement rattachés, un des carbones du cycle étant optionnellement remplacé par un atome d'oxygène, de soufre ou par un atome d'azote substitué par un radical R8 ; m et R8 ayant les significations données ci-après,
R5' représente un atome d'hydrogène, un fluor, un radical alkyle comportant de 1 à 5 atomes de carbone inclus ou un radical alkyle fluoré ou perfluoré comportant de 1 à 5 atomes de carbone,
R6 représente un atome d'hydrogène, un radical -COOtBu ou un radical -COOBn,
R7 représente un hydrogène, un alkyle de C1 à C3, un halogène, -CF3, -COR9, -OR9, - NR9R10, -NO2, -CN, -SO2R9, -S(O)R9, -S(=O)(=NR9)R10', -SO2NR9R10, - NR9SO2R10, -NR9COR10, -NR9CO2R10, -CONR9R10, ou -CO2R9,
R7a représente un hydrogène ou un alkyle de C1 à C5,
R8 représente un hydrogène, -OH, -SO2R9, -COR9, -CO2R9, un aryle, un heteroaryle, un arylalkyle, un heteroarylalkyle, un alkyle, un cycloalkyle ou encore un cycloalkylalkyle,
R9 et R10 sont identiques ou différents et sont indépendamment choisis dans le groupe constitué par un hydrogène, un aryle, un heteroaryle, un arylalkyle, un heteroarylalkyle, un alkyle, un cycloalkyle ou un cycloalkylalkyle,
ou encore
R9 et R10 peuvent être liés entre eux lorsqu'ils sont portés par un même atome d'azote de façon à former un hétérocycle ayant entre 3 et 7 chainons et comportant un ou deux hétéroatomes choisis parmi l'oxygène , le soufre et l'azote en plus de l'atome d'azote auquel ils sont rattachés.
R10' représente un aryle, un heteroaryle, un arylalkyle, un heteroarylalkyle, un alkyle, un cycloalkyle ou un cycloalkylalkyle,
R11 et R12, identiques ou différents, représentent un hydrogène, un alkyle de C1 à C5, un cycloalkyle de C3 à C6, une chaine -(CH₂)ₚ- formant un cycle contenant de 4 à 6 atomes avec l'atome d'azote auquel ils sont rattachés,
X et X', identiques ou différents, représentent un atome d'oxygène, un atome de soufre, ou un atome d'azote substitué par un radical R6,
Y représente un atome d'oxygène, un atome de soufre, ou un atome d'azote substitué par un radical R8,
Z représente un atome de carbone ou un atome d'azote,
m = 3, 4, 5, ou 6 et
p = 3, 4, ou 5.

2. Composé selon la revendication 1, **caractérisé en ce que** dans la formule (I) précitée:
A représente 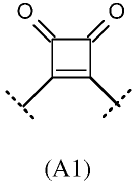
B représente 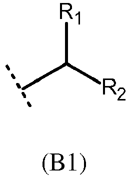
R1 représente un hydrogène, un alkyle de C1 à C5, un cycloalkyle de C3 à C8, un cycloalkyle répondant à la formule (1') suivante dans laquelle X a la signification donnée ci-après : 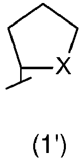
R2 représente :
- un alkyle de C1 à C5, non substitué ou substitué par un ou plusieurs groupes choisis parmi F, étant entendu que lorsque le radical alkyle de C1 à C5 n'est substitué que par un ou plusieurs atomes de fluor, il s'agit d'un radical alkyle fluoré ou d'un radical alkyle perfluoré de C1 à C5,
- un alkyle de C1 à C5 dans lequel un atome de carbone est remplacé par un atome d'oxygène,
- un alcyne de C2 à C5, non substitué ou substitué par un ou plusieurs groupes choisis parmi le fluor et le phényle,
- un cycle aromatique ou heteroaromatique sélectionné dans le groupe constitué par les cycles répondant aux formules (a), (b1) et (d1) suivantes dans lesquelles R7 et Z ont les significations données ci-après: 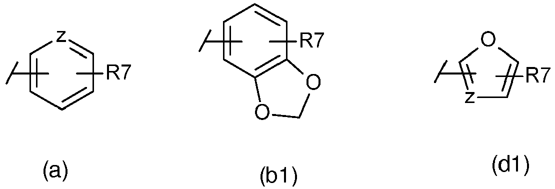 R7 peut être présent plusieurs fois sur un cycle, et au maximum, autant de fois qu'il y a d'atomes substituables. Les significations de chaque substituant R7 peuvent être identiques ou différentes,
R3 représente un hydrogène ou un chlore,
R4 et R5, identiques ou différents, représentent un hydrogène, un alkyle de C1 à C3, un alkyle de C1 à C8 dans lequel un atome de carbone est remplacé par un atome d'oxygène,
R7 représente un hydrogène, un alkyle de C1 à C3 ou un fluor,
X représente un atome de soufre, et
Z représente un atome de carbone ou un atome d'azote.

3. Composé selon la revendication 1 ou 2, **caractérisé en ce que** dans la formule (I) précitée:
A représente 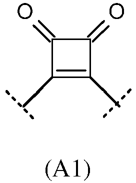
B représente 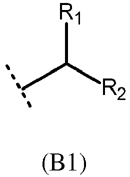
R1 représente un alkyle de C1 à C5,
R2 représente :
- un alkyle de C1 à C5,
- un alcyne de C2 à C5, substitué par un ou plusieurs groupes choisis parmi le fluor et le phényle,
- un cycle aromatique ou heteroaromatique sélectionné dans le groupe constitué par les cycles répondant aux formules (a), (b1) et (d1) suivantes dans lesquelles R7 et Z ont les significations données ci-après: 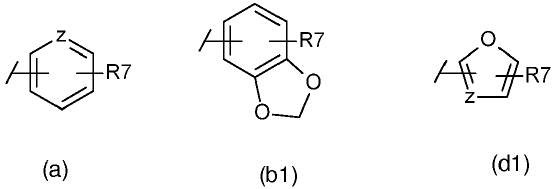 R7 peut être présent plusieurs fois sur un cycle, et au maximum, autant de fois qu'il y a d'atomes substituables. Les significations de chaque substituant R7 peuvent être identiques ou différentes,
R3 représente un hydrogène ou un chlore,
R4 et R5, identiques ou différents, représentent un hydrogène ou un alkyle de C1 à C3,
R7 représente un hydrogène, un alkyle de C1 à C3 ou un fluor, et
Z représente un atome de carbone ou un atome d'azote.

4. Composé selon la revendication 1, choisi parmi:
Composé 1: 3-(4-Chloro-2-hydroxy-3-methanesulfoxymin-phenylamino)-4-[(R)-1-(5-methyl-furan-2-yl)-propylamino]-cyclobut-3-ene-1,2-dione
Composé 2: 3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione
Composé 3: 3-{4-Chloro-2-hydroxy-3-methane-[(N- pyridin-4-yl)-sulfoximin]-phenylamino}-4-[(R)-1-(5-methyl-furan-2-yl)-propylamino]-cyclobut-3-ene-1,2-dione
Composé 4: 3-{4-Chloro-2-hydroxy-3-methane-[(N-pyridin-4-yl)-sulfoximin]-phenylamino}-4-(1-Ethyl-propylamino)-cyclobut-3-ene-1,2-dione
Composé 5: 3-{4-Chloro-2-hydroxy-3-methane-[(N-methyl)-sulfoximin]-phenylamino}-4-{[(S)-(5-methyl-furan-2-yl)-(R)-tetrahydro-thiophen-2-yl-methyl]-amino}-cyclobut-3-ene-1,2-dione
Composé 6: 1-(2-chloro-3-fluoro-phenyl)-3-(4-chloro-2-hydroxy-3-methanesulfoximine-phenyl)-urea
Composé 7: 1-(2-Chloro-3-fluoro-phenyl)-3-{4-chloro-2-hydroxy-3-methanes-[(N-methyl)-sulfoximin]-phenyl}-urea
Composé 8: 3-{2-hydroxy-3-methane-[(N-methyl)-sulfoximin]-phenylamino}-4-[(R)-1-(5-methyl-furan-2-yl)-propylamino]-cyclobut-3-ene-1,2-dione
Composé 9: 3-[4-chloro-3-[N-(2-dimethylaminoethyl)-S-methyl-sulfonimidoyl]-2-hydroxy-anilino]-4-ethoxy-cyclobut-3-ene-1,2-dione
Composé 10: 3-[4-chloro-3-[N-(2-dimethylaminoethyl)-S-methyl-sulfonimidoyl]-2-hydroxy-anilino]-4-(1-ethylpropylamino)cyclobut-3-ene-1,2-dione
Composé 11: 3-[4-chloro-2-hydroxy-3-[N-(2-methoxyethyl)-S-methyl-sulfonimidoyl]anilino]-4-[[(1R)-1-(5-methyl-2-furyl)propyl]amino]cyclobut-3-ene-1,2-dione
Composé 12: (-)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione
Composé 13: (+)-3-{4-Chloro-2-hydroxy-3-methane-[(N-methyl)-sulfoximin]-phenylamino}-4-[(R)-1-(pyridin-2-yl)-propylamino]-cyclobut-3-ene-1,2-dione
Composé 14: (-)-3-{2-Hydroxy-3-methane-[(N-methyl)-sulfoximin]-phenylamino}-4-[(R)-1-(pyridin-2-yl)-propylamino]-cyclobut-3-ene-1,2-dione
Composé 15: (-)-3-{2-Hydroxy-3-methane-[(N-methyl)-sulfoximin]-phenylamino}-4-[(R)-1-(pyridin-2-yl)-propylamino]-cyclobut-3-ene-1,2-dione
Composé 16: (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione
Composé 17: (+)-3-{2-Hydroxy-3-methane-[(N-methyl)-sulfoximin]-phenylamino}-4-[(R)-1-(5-methyl-furan-2-yl)-propylamino]-cyclobut-3-ene-1,2-dione
Composé 18: (-)-3-(2-Hydroxy-3-methane-[(N-methyl)-sulfoxim]-phenylamino-[4-(1-ethyl-propylamino]-cyclobut-3-ene-1,2-dione
Composé 19: (+)-3-(2-Hydroxy-3-methane-[(N-methyl)-sulfoxim]-phenylamino-[4-(1-ethyl-propylamino]-cyclobut-3-ene-1,2-dione
Composé 20: (+)-3-{4-Chloro-2-hydroxy-3-methane-[(N-methyl)-sulfoximin]-phenylamino}-4-[(R)-1-(5-methyl-furan-2-yl)-propylamino]-cyclobut-3-ene-1,2-dione.

5. Composition pharmaceutique comprenant une quantité efficace d'un composé ou d'un sel pharmaceutiquement acceptable dudit composé selon l'une des revendications 1 à 4 en association avec un solvant ou un support pharmaceutiquement acceptable.

6. Composé selon l'une des revendications 1-4 ou composition pharmaceutique selon la revendication 5 pour son utilisation en tant que médicament.

7. Composé selon l'une des revendications 1-4 ou composition pharmaceutique selon la revendication 5 pour son utilisation dans le traitement de maladies médiées par les α-chimiokines choisies dans le groupe comprenant les dermatoses neutrophiliques, notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

8. Composé ou composition pharmaceutique selon la revendication 7 pour son utilisation dans le traitement de maladies dermatologiques telles que les dermatoses neutrophiliques, notamment le psoriasis, les dermatites atopiques, l'acné et la rosacée.

## Patentansprüche

1. Verbindung, die der folgenden allgemeinen Formel (I) entspricht, oder ein Salz davon oder ein Enantiomer davon: 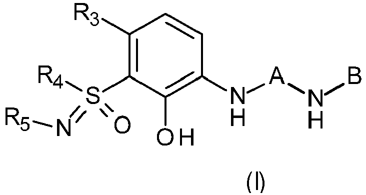 in der:
A für 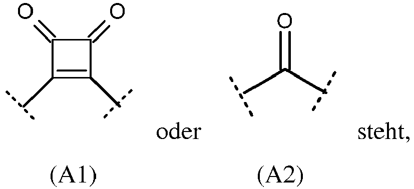
B für 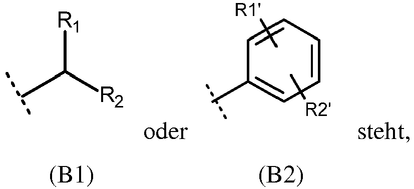
wobei
R1' und R2', die identisch oder verschieden sind, für einen Wasserstoff, ein Halogen, ein C1- bis C5-Alkyl stehen, das mit einem oder mehreren Fluoratomen, einem C1- bis C5-Alkoxy, OCF3, OH, CN, NR11R12 substituiert sein kann,
R1 und R2, die identisch oder verschieden sind, stehen für:
- einen Wasserstoff,
- ein C1- bis C5-Alkyl, das mit einer oder mehreren Gruppen, ausgewählt aus F, OH, OCH3 und NR11R12, substituiert sein kann; wobei R11 und R12 die nachstehend gegebene Bedeutung besitzen, wenn allerdings der C1- bis C5-Alkylrest nur mit einem oder mehreren Fluoratomen substituiert ist, dann handelt es sich um einen fluorierten C1- bis C5-Alkylrest oder einen perfluorierten C1- bis C5-Alkylrest,
- ein C1- bis C5-Alkyl, in dem ein Kohlenstoffatom durch ein Sauerstoffatom oder durch ein Schwefelatom ersetzt ist, wobei besagtes C1- bis C5-Alkyl mit einer oder mehreren Gruppen, ausgewählt aus F, OH und NR11R12, substituiert sein kann; wobei R11 und R12 die nachstehend gegebene Bedeutung besitzen,
- ein C3- bis C8-Cycloalkyl,
- ein C2- bis C5-Alkin, das mit einer oder mehreren Gruppen, ausgewählt aus F, OH, Phenyl, NR11R12, substituiert sein kann; wobei R11 und R12 die nachstehend gegebene Bedeutung besitzen,
- ein Cycloalkyl, das einer der folgenden Formeln (1), (2), (3), (4), (5) oder (6) entspricht, in denen R5', X und X' die nachstehend gegebenen Bedeutungen besitzen: 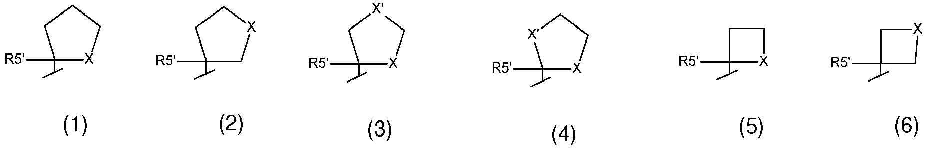
- einen heteroaromatischen oder aromatischen Ring, ausgewählt aus der Gruppe, bestehend aus den Ringen, die den folgenden Formeln (a) bis (o) entsprechen, in denen R7, R7a, Y und Z die nachstehend gegebenen Bedeutungen besitzen: 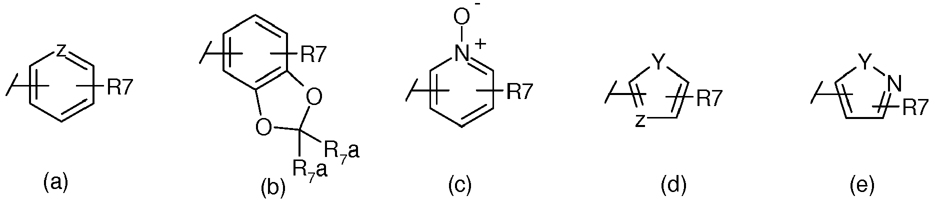 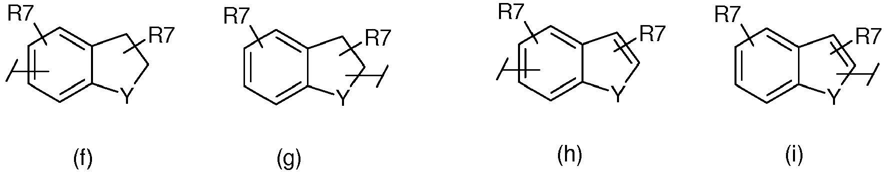 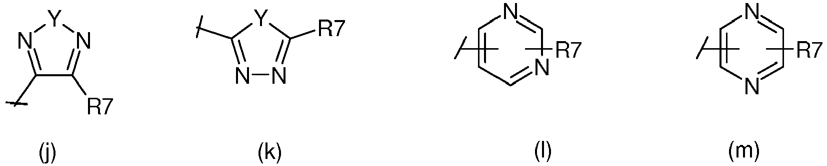 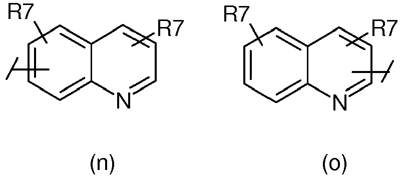 R7 mehrfach an einem Ring vorhanden sein kann und maximal so oft, wie es substituierbare Atome gibt. Die Bedeutungen jedes Substituenten R7 können identisch oder verschieden sein,
R3 für einen Wasserstoff, ein Halogen, ein C1- bis C5-Alkyl, ein C1- bis C5-Alkoxy, -CF3, - OCF3, -OH, -NO2, -CN steht,
R4 und R5, die identisch oder verschieden sind, stehen für:
- einen Wasserstoff,
- ein C1- bis C8-Alkyl, das mit einer oder mehreren Gruppen, ausgewählt aus F, OH, NR11R12, substituiert sein kann, wobei R11 und R12 die nachstehend gegebene Bedeutung besitzen,
- ein C1- bis C8-Alkyl, in dem ein Kohlenstoffatom durch ein Stickstoffatom, durch ein Sauerstoffatom oder durch ein Schwefelatom ersetzt ist, wobei besagtes C1- bis C8-Alkyl mit einer oder mehreren Gruppen, ausgewählt aus F, OH und NR11R12, substituiert sein kann; wobei R11 und R12 die nachstehend gegebene Bedeutung besitzen,
- ein C3- bis C8-Cycloalkyl,
- ein C3- bis C8-Cycloalkyl, bei dem eines der Kohlenstoffatome durch ein Sauerstoffatom oder durch ein Stickstoffatom, substituiert mit einem Rest R7a, ersetzt ist,
- ein Heterocycloalkyl mit 5 bis 7 Ringatomen,
- ein Cycloalkylalkyl, wobei das Cycloalkyl ein C3- bis C8-Cycloalkyl ist und das Alkyl ein C1- bis C8-Alkyl ist,
- ein Phenyl,
- ein Phenyl, substituiert mit einem Rest R7,
- ein Heteroaryl,
- ein Arylalkyl, wobei das Alkyl ein C1- bis C5-Alkyl ist,
- ein Heteroarylalkyl, wobei das Alkyl ein C1- bis C5-Alkyl ist,
oder auch
R4 und R5 für eine -(CH₂)ₘ-Kette stehen und einen Ring mit 5 bis 8 Atomen mit den Schwefel- und Stickstoffatomen bilden, an die sie jeweils gebunden sind, wobei einer der Ringkohlenstoffe gegebenenfalls durch ein Sauerstoffatom, Schwefelatom oder durch ein Stickstoffatom, substituiert mit einem Rest R8, ersetzt ist, wobei m und R8 die nachstehend gegebenen Bedeutungen besitzen,
R5' für ein Wasserstoffatom, ein Fluoratom, einen 1 bis einschließlich 5 Kohlenstoffatome umfassenden Alkylrest oder einen 1 bis 5 Kohlenstoffatome umfassenden perfluorierten oder fluorierten Alkylrest steht,
R6 für ein Wasserstoffatom, einen -COOtBu-Rest oder einen -COOBn-Rest steht,
R7 für einen Wasserstoff, ein C1- bis C3-Alkyl, ein Halogen, -CF3, -COR9, -OR9, -NR9R10, - NO2, -CN, -SO2R9, -S(O)R9, -S(=O)(=NR9)R10', -SO2NR9R10, - NR9SO2R10, - NR9COR10, -NR9CO2R10, -CONR9R10 oder -CO2R9 steht,
R7a für einen Wasserstoff oder ein C1- bis C5-Alkyl steht,
R8 für einen Wasserstoff, -OH, SO2R9, -COR9, -CO2R9, ein Aryl, ein Heteroaryl, ein Arylalkyl, ein Heteroarylalkyl, ein Alkyl, ein Cycloalkyl oder auch ein Cycloalkylalkyl steht,
R9 und R10 identisch oder verschieden sind und unabhängig voneinander aus der Gruppe ausgewählt sind, bestehend aus einem Wasserstoff, einem Aryl, einem Heteroaryl, einem Arylalkyl, einem Heteroarylalkyl, einem Alkyl, einem Cycloalkyl oder einem Cycloalkylalkyl,
oder auch
R9 und R10 miteinander verbunden sein können, wenn sie von demselben Stickstoffatom getragen sind, um einen Heterocyclus mit 3 bis 7 Gliedern zu bilden, und ein oder zwei aus Sauerstoff, Schwefel und Stickstoff ausgewählte Heteroatome zuzüglich zu dem Stickstoffatom, an das sie gebunden sind, umfassen,
R10' für ein Aryl, ein Heteroaryl, ein Arylalkyl, ein Heteroarylalkyl, ein Alkyl, ein Cycloalkyl oder ein Cycloalkylalkyl steht,
R11 und R12, die identisch oder verschieden sind, für einen Wasserstoff, ein C1- bis C5-Alkyl, ein C3- bis C6-Cycloalkyl, eine -(CH₂)ₚ-Kette stehen und einen Ring mit 4 bis 6 Atomen mit dem Stickstoffatom, an das sie gebunden sind, bilden,
X und X', die identisch oder verschieden sind, für ein Sauerstoffatom, ein Schwefelatom oder ein Stickstoffatom, substituiert mit einem Rest R6, stehen,
Y für ein Sauerstoffatom, ein Schwefelatom oder ein Stickstoffatom, substituiert mit einem Rest R8, steht,
Z für ein Kohlenstoffatom oder ein Stickstoffatom steht,
m = 3, 4, 5 oder 6 und
p = 3, 4 oder 5.

2. Verbindung gemäß Anspruch 1, **dadurch gekennzeichnet, dass** in der vorerwähnten Formel (I):
A für 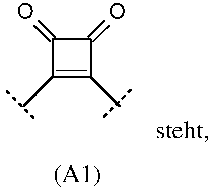
B für 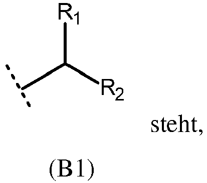
R1 für einen Wasserstoff, ein C1- bis C5-Alkyl, ein C3- bis C8-Cycloalkyl, ein Cycloalkyl steht, das der folgenden Formel (1') entspricht, in der X die nachstehend gegebene Bedeutung besitzt: 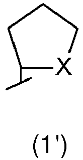
R2 steht für:
- ein C1- bis C5-Alkyl, das mit einer oder mehreren Gruppen, ausgewählt aus F, substituiert sein kann, wenn allerdings der C1- bis C5-Alkylrest nur mit einem oder mehreren Fluoratomen substituiert ist, dann handelt es sich um einen fluorierten C1- bis C5-Alkylrest oder einen perfluorierten C1- bis C5-Alkylrest,
- ein C1- bis C5-Alkyl, in dem ein Kohlenstoffatom durch ein Sauerstoffatom ersetzt ist,
- ein C2- bis C5-Alkin, das mit einer oder mehreren Gruppen, ausgewählt aus Fluor und Phenyl, substituiert sein kann,
- einen heteroaromatischen oder aromatischen Ring, ausgewählt aus der Gruppe, bestehend aus den Ringen, die den folgenden Formeln (a), (b1) und (d1) entsprechen, in denen R7 und Z die nachstehend gegebenen Bedeutungen besitzen: 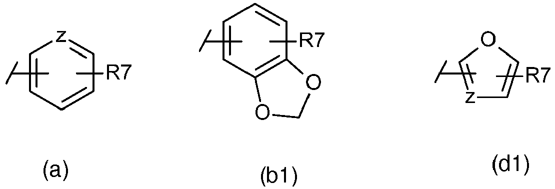 R7 mehrfach an einem Ring vorhanden sein kann und maximal so oft, wie es substituierbare Atome gibt. Die Bedeutungen jedes Substituenten R7 können identisch oder verschieden sein,
R3 für einen Wasserstoff oder ein Chlor steht,
R4 und R5, die identisch oder verschieden sind, für einen Wasserstoff, ein C1- bis C3-Alkyl, ein C1- bis C8-Alkyl stehen, in dem ein Kohlenstoffatom durch ein Sauerstoffatom ersetzt ist,
R7 für einen Wasserstoff, ein C1- bis C3-Alkyl oder ein Fluor steht,
X für ein Schwefelatom steht, und
Z für ein Kohlenstoffatom oder ein Stickstoffatom steht.

3. Verbindung gemäß Anspruch 1 oder 2, **dadurch gekennzeichnet, dass** in der vorerwähnten Formel (I):
A für 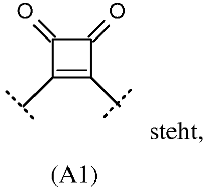
B für 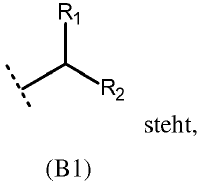
R1 für ein C1- bis C5-Alkyl steht,
R2 steht für:
- ein C1- bis C5-Alkyl,
- ein C2- bis C5-Alkin, das mit einer oder mehreren Gruppen, ausgewählt aus Fluor und Phenyl, substituiert sein kann,
- einen heteroaromatischen oder aromatischen Ring, ausgewählt aus der Gruppe, bestehend aus den Ringen, die den folgenden Formeln (a), (b1) und (d1) entsprechen, in denen R7 und Z die nachstehend gegebenen Bedeutungen besitzen: 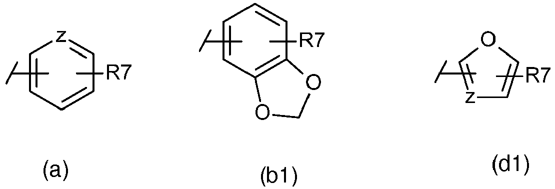 R7 mehrfach an einem Ring vorhanden sein kann und maximal so oft, wie es substituierbare Atome gibt. Die Bedeutungen jedes Substituenten R7 können identisch oder verschieden sein,
R3 für einen Wasserstoff oder ein Chlor steht,
R4 und R5, die identisch oder verschieden sind, für einen Wasserstoff, ein C1- bis C3-Alkyl stehen,
R7 für einen Wasserstoff, ein C1- bis C3-Alkyl oder ein Fluor steht, und
Z für ein Kohlenstoffatom oder ein Stickstoffatom steht.

4. Verbindung gemäß Anspruch 1, ausgewählt aus:
| | |
|---|---|
| Verbindung 1: | 3-(4-Chlor-2-hydroxy-3-methansulfoximin-phenylamino)-4-[(R)-1-(5-methylfuran-2-yl)-propylamino]-cyclobut-3-en-1,2-dion |
| Verbindung 2: | 3-{4-Chlor-2-hydroxy-3-methan-[(N-methyl)-sulfoximin]-phenylamino}-4-[(R)-1-(5-methyl-furan-2-yl)-propylamino]-cyclobut-3-en-1,2-dion |
| Verbindung 3: | 3-{4-Chlor-2-hydroxy-3-methan-[(N-pyridin-4-yl)-sulfoximin]-phenylamino}-4-[(R)-1-(5-methyl-furan-2-yl)-propylamino]-cyclobut-3-en-1,2-dion |
| Verbindung 4: | 3-{4-Chlor-2-hydroxy-3-methan-[(N-pyridin-4-yl)-sulfoximin]-phenylamino}-4-(1-ethyl-propylamino)-cyclobut-3-en-1,2-dion |
| Verbindung 5: | 3-{4-Chlor-2-hydroxy-3-methan-[(N-methyl)-sulfoximin]-phenylamino}-4-{[(S)-(5-methyl-furan-2-yl)-(R)-tetrahydro-thiophen-2-yl-methyl]-amino}-cyclobut-3-en-1,2-dion |
| Verbindung 6: | 1-(2-Chlor-3-fluor-phenyl)-3-(4-chlor-2-hydroxy-3-methansulfoximin-phenyl)-carbamid |
| Verbindung 7: | 1-(2-Chlor-3-fluor-phenyl)-3-{4-chlor-2-hydroxy-3-methan-[(N-methyl)-sulfoximin]-phenyl}-carbamid |
| Verbindung 8: | 3-{2-Hydroxy-3-methan-[(N-methyl)-sulfoximin]-phenylamino}-4-[(R)-1-(5-methyl-furan-2-yl)-propylamino]-cyclobut-3-en-1,2-dion |
| Verbindung 9: | 3-[4-Chlor-3-[N-(dimethylaminoethyl)-S-methyl-sulfonimidoyl]-2-hydroxy-anilino]-4-ethoxy-cyclobut-3-en-1,2-dion |
| Verbindung 10: | 3-[4-Chlor-3-[N-(2-dimethylaminoethyl)-S-methyl-sulfonimidoyl]-2-hydroxyanilino]-4-(1-ethylpropylamino)-cyclobut-3-en-1,2-dion |
| Verbindung 11: | 3-[4-Chlor-2-hydroxy-3-[N-(2-methoxyethyl)-S-methylsulfonimidoyl]anilino]-4-[[1(R)-1-(5-methyl-2-furyl)propyl]amino]-cyclobut-3-en-1,2-dion |
| Verbindung 12: | (-)-3-{4-Chlor-2-hydroxy-3-methan-[(N-methyl)-sulfoximin]-phenylamino}-4-[(R)-1-(pyridin-2-yl)-propylamino]-cyclobut-3-en-1,2-dion |
| Verbindung 13: | (+)-3-{4-Chlor-2-hydroxy-3-methan-[(N-methyl)-sulfoximin]-phenylamino}-4-[(R)-1-(pyridin-2-yl)-propylamino]-cyclobut-3-en-1,2-dion |
| Verbindung 14: | (-)-3-{2-Hydroxy-3-methan-[(N-methyl)-sulfoximin]-phenylamino}-4-[(R)-1-(pyridin-2-yl)-propylamino]-cyclobut-3-en-1,2-dion |
| Verbindung 15: | (-)-3-{2-Hydroxy-3-methan-[(N-methyl)-sulfoximin]-phenylamino}-4-[(R)-1-(pyridin-2-yl)-propylamino]-cyclobut-3-en-1,2-dion |
| Verbindung 16: | (+)-3-{2-Hydroxy-3-methan-[(N-methyl)-sulfoximin]-phenylamino}-4-[(R)-1-(5-methyl-furan-2-yl)-propylamino]-cyclobut-3-en-1,2-dion |
| Verbindung 17: | (+)-3-{2-Hydroxy-3-methan-[(N-methyl)-sulfoximin]-phenylamino}-4-[(R)-1-(5-methyl-furan-2-yl)-propylamino]-cyclobut-3-en-1,2-dion |
| Verbindung 18: | (-)-3-(2-Hydroxy-3-methan-[(N-methyl)-sulfoxim]-phenylamino-[4-(1-ethyl-propylamino]-cyclobut-3-en- 1,2-dion |
| Verbindung 19: | (+)-3-(2-Hydroxy-3-methan-[(N-methyl)-sulfoxim]-phenylamino-[4-(1-ethyl-propylamino]-cyclobut-3-en-1,2-dion |
| Verbindung 20: | (+)-3-{4-Chlor-2-hydroxy-3-methan-[(N-methyl)-sulfoximin]-phenylamino}-4-[(R)-1-(5-methyl-furan-2-yl)-propylamino]-cyclobut-3-en-1,2-dion. |

5. Pharmazeutische Zusammensetzung, umfassend eine wirksame Menge einer Verbindung oder eines pharmazeutisch verträglichen Salzes besagter Verbindung gemäß einem der Ansprüche 1 bis 4 in Kombination mit einem pharmazeutisch verträglichen Träger oder Lösemittel.

6. Verbindung gemäß einem der Ansprüche 1 bis 4 oder pharmazeutische Zusammensetzung gemäß Anspruch 5 zur Verwendung als Medikament.

7. Verbindung gemäß einem der Ansprüche 1 bis 4 oder pharmazeutische Zusammensetzung gemäß Anspruch 5 zur Verwendung in der Behandlung von α-Chemokin-vermittelten Krankheiten, ausgewählt aus der Gruppe, umfassend neutrophile Dermatosen, insbesondere Psorias, atopische Dermatitiden, Akne, Rosacea, Asthma, chronische obstruktive Lungenkrankheiten, Atemwegserkrankungen bei Erwachsenen, Arthritis, entzündliche Darmkrankheiten, Morbus Crohn, Transplantatabstoßung, Mukoviszidose und Hautkrebse.

8. Verbindung oder pharmazeutische Zusammensetzung gemäß Anspruch 7 zur Verwendung in der Behandlung von Hautkrankheiten wie neutrophilen Dermatosen, insbesondere Psorias, atopischen Dermatitiden, Akne und Rosacea.

## Claims

1. A compound corresponding to the general formula (I) below, or a salt thereof or an enantiomer thereof: 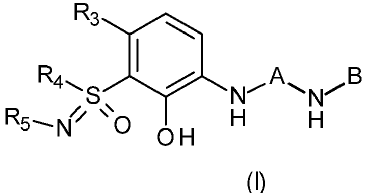 wherein:
A represents 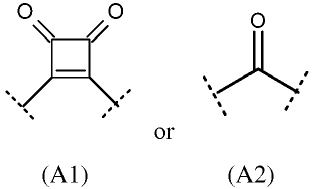
B represents 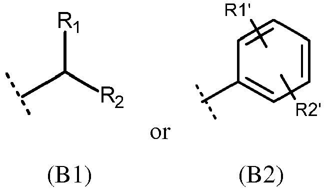 with
R1' and R2', which may be identical or different, represent a hydrogen, a halogen, a C1 to C5 alkyl which is unsubstituted or substituted with one or more fluorine atoms, a C1 to C5 alkoxy, OCF3, OH, CN, NR11R12,
R1 and R2, which may be identical or different, represent:
- a hydrogen,
- a C1 to C5 alkyl, which is unsubstituted or substituted with one or more groups chosen from F, OH, OCH3 and NR11R12; R11 and R12 having the meaning given below, it being understood that when the C1 to C5 alkyl radical is substituted only with one or more fluorine atoms, it is a C1 to C5 fluoroalkyl radical or perfluoroalkyl radical,
- a C1 to C5 alkyl in which a carbon atom is replaced with an oxygen atom or with a sulfur atom, said C1 to C5 alkyl being unsubstituted or substituted with one or more groups chosen from F, OH and NR11R12, R11 and R12 having the meaning given below,
- a C3 to C8 cycloalkyl radical,
- a C2 to C5 alkyne, which is unsubstituted or substituted with one or more groups chosen from F, OH, phenyl, NR11R12, R11 and R12 having the meaning given below,
- a cycloalkyl corresponding to one of the formulae (1), (2), (3), (4), (5) or (6) below in which R5', X and X' have the meanings given below: 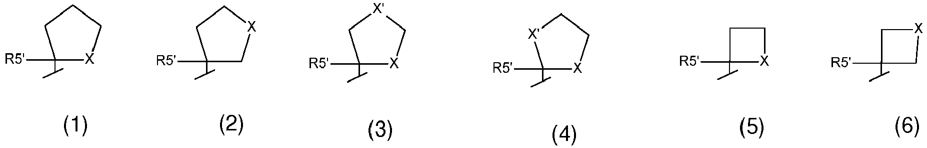
- an aromatic or heteroaromatic ring selected from the group consisting of the rings corresponding to formulae (a) to (o) below in which R7, R7a, Y and Z have the meanings given below: 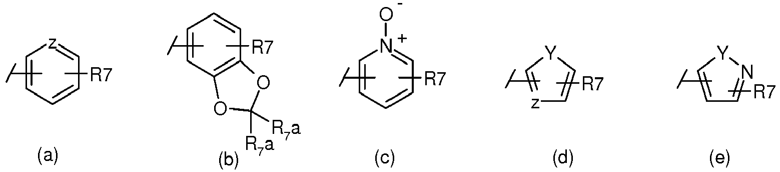 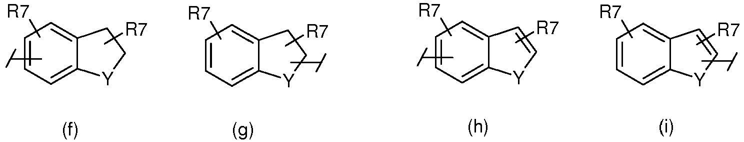 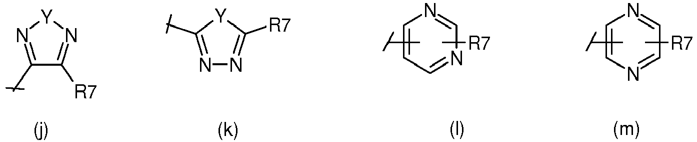 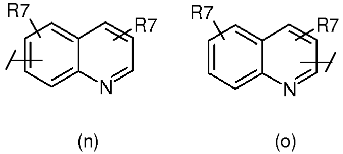
R7 may be present several times on a ring, and at most as many times as there are substitutable atoms; the meanings of each substituent R7 may be identical or different,
R3 represents a hydrogen, a halogen, a C1 to C5 alkyl, a C1 to C5 alkoxy, -CF3, -OCF3, -OH, -NO2 or -CN,
R4 and R5, which may be identical or different, represent:
- a hydrogen,
- a C1 to C8 alkyl, which is unsubstituted or substituted with one or more groups chosen from F, OH, NR11R12, R11 and R12 having the meaning given below,
- a C1 to C8 alkyl in which a carbon atom is replaced with a nitrogen atom, with an oxygen atom or with a sulfur atom, said C1 to C8 alkyl being unsubstituted or substituted with one or more groups chosen from F, OH, NR11R12, R11 and R12 having the meaning given below,
- a C3 to C8 cycloalkyl radical,
- a C3 to C8 cycloalkyl radical, one of the carbon atoms of which is replaced with an oxygen atom or with a nitrogen atom substituted with a radical R7a,
- a heterocycloalkyl of 5 to 7 ring atoms,
- a cycloalkylalkyl, the cycloalkyl being C3 to C8 and the alkyl C1 to C8,
- a phenyl,
- a phenyl substituted with a radical R7,
- a heteroaryl,
- an arylalkyl, the alkyl being C1-C5,
- a heteroarylalkyl, the alkyl being C1-C5,
or alternatively
R4 and R5 represent a chain -(CH₂)ₘ- forming a ring containing from 5 to 8 atoms with the sulfur and nitrogen atoms to which they are respectively attached, one of the carbons of the ring being optionally replaced with an oxygen or sulfur atom or with a nitrogen atom substituted with a radical R8; m and R8 having the meanings given below,
R5' represents a hydrogen atom, a fluorine, an alkyl radical comprising from 1 to 5 carbon atoms inclusive or a fluoroalkyl or perfluoroalkyl radical comprising from 1 to 5 carbon atoms,
R6 represents a hydrogen atom, a radical -COOtBu or a radical -COOBn,
R7 represents a hydrogen, a C1 to C3 alkyl, a halogen, -CF3, -COR9, -OR9, -NR9R10, -NO2, -CN, -SO2R9, -S(O)R9, -S(=O)(=NR9)R10', -SO2NR9R10, -NR9SO2R10, -NR9COR10, -NR9CO2R10, -CONR9R10 or -CO2R9,
R7a represents a hydrogen or a C1 to C5 alkyl,
R8 represents a hydrogen, -OH, -SO2R9, -COR9, -CO2R9, an aryl, a heteroaryl, an arylalkyl, a heteroarylalkyl, an alkyl, a cycloalkyl or alternatively a cycloalkylalkyl,
R9 and R10 are identical or different and are chosen independently from the group consisting of a hydrogen, an aryl, a heteroaryl, an arylalkyl, a heteroarylalkyl, an alkyl, a cycloalkyl, and a cycloalkylalkyl,
or alternatively
R9 and R10 may be linked together when they are borne by the same nitrogen atom so as to form a 3- to 7-membered heterocycle comprising one or two heteroatoms chosen from oxygen, sulfur and nitrogen in addition to the nitrogen atom to which they are attached,
R10' represents an aryl, a heteroaryl, an arylalkyl, a heteroarylalkyl, an alkyl, a cycloalkyl or a cycloalkylalkyl,
R11 and R12, which may be identical or different, represent a hydrogen, a C1 to C5 alkyl, a C3 to C6 cycloalkyl, or a chain -(CH₂)ₚ- forming a ring containing from 4 to 6 atoms with the nitrogen atom to which they are attached,
X and X', which may be identical or different, represent an oxygen atom, a sulfur atom or a nitrogen atom substituted with a radical R6,
Y represents an oxygen atom, a sulfur atom or a nitrogen atom substituted with a radical R8,
Z represents a carbon atom or a nitrogen atom,
m = 3, 4, 5 or 6 and
p = 3, 4 or 5.

2. The compound according to Claim 1, wherein in the abovementioned formula (I):
A represents 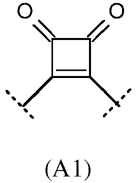
B represents 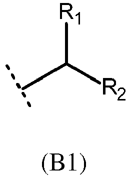
R1 represents a hydrogen, a C1 to C5 alkyl, a C3 to C8 cycloalkyl radical, or a cycloalkyl corresponding to formula (1') below in which X has the meaning given below: 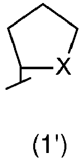
R2 represents:
- a C1 to C5 alkyl, which is unsubstituted or substituted with one or more groups chosen from F, it being understood that when the C1 to C5 alkyl radical is substituted only with one or more fluorine atoms, it is a C1 to C5 fluoroalkyl radical or perfluoroalkyl radical,
- a C1 to C5 alkyl in which a carbon atom is replaced with an oxygen atom,
- a C2 to C5 alkyne, which is unsubstituted or substituted with one or more groups chosen from fluorine and phenyl,
- an aromatic or heteroaromatic ring selected from the group consisting of the rings corresponding to formulae (a), (b1) and (d1) below in which R7 and Z have the meanings given below: 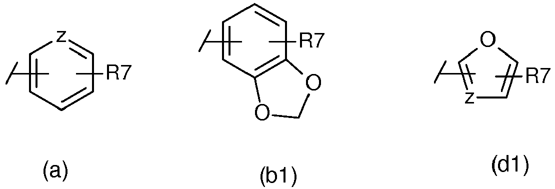 R7 may be present several times on a ring, and at most as many times as there are substitutable atoms; the meanings of each substituent R7 may be identical or different,
R3 represents a hydrogen or a chlorine,
R4 and R5, which may be identical or different, represent a hydrogen, a C1 to C3 alkyl, or a C1 to C8 alkyl in which a carbon atom is replaced with an oxygen atom,
R7 represents a hydrogen, a C1 to C3 alkyl or a fluorine,
X represents a sulfur atom, and
Z represents a carbon atom or a nitrogen atom.

3. The compound according to Claim 1 or 2, wherein in the abovementioned formula (I):
A represents 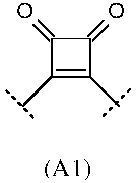
B represents 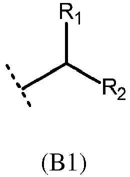
R1 represents a C1 to C5 alkyl,
R2 represents:
- a C1 to C5 alkyl,
- a C2 to C5 alkyne, substituted with one or more groups chosen from fluorine and phenyl,
- an aromatic or heteroaromatic ring selected from the group consisting of the rings corresponding to formulae (a), (b1) and (d1) below in which R7 and Z have the meanings given below: 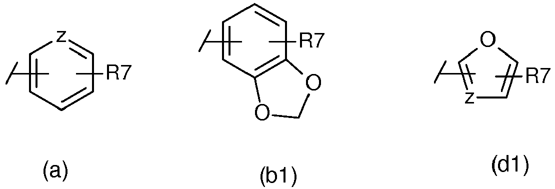 R7 may be present several times on a ring, and at most as many times as there are substitutable atoms; the meanings of each substituent R7 may be identical or different,
R3 represents a hydrogen or a chlorine,
R4 and R5, which may be identical or different, represent a hydrogen or a C1 to C3 alkyl,
R7 represents a hydrogen, a C1 to C3 alkyl or a fluorine, and
Z represents a carbon atom or a nitrogen atom.

4. The compound according to Claim 1, chosen from:
Compound 1: 3-(4-chloro-2-hydroxy-3-methanesulfoximinophenylamino)-4-[(R)-1-(5-methylfuran-2-yl)propylamino]cyclobut-3-ene-1,2-dione
Compound 2: 3-{4-chloro-2-hydroxy-3-methane[(N-methyl)sulfoximino]-phenylamino}-4-[(R)-1-(5-methylfuran-2-yl)propylamino]cyclobut-3-ene-1,2-dione
Compound 3: 3-{4-chloro-2-hydroxy-3-methane[(N-pyridin-4-yl)sulfoximino]-phenylamino}-4-[(R)-1-(5-methylfuran-2-yl)propylamino]cyclobut-3-ene-1,2-dione
Compound 4: 3-{4-chloro-2-hydroxy-3-methane[(N-pyridin-4-yl)sulfoximino]-phenylamino}-4-(1-ethylpropylamino)cyclobut-3-ene-1,2-dione
Compound 5: 3-{4-chloro-2-hydroxy-3-methane[(N-methyl)sulfoximino]-phenylamino}-4-{[(S)-(5-methylfuran-2-yl)-(R)-tetrahydrothiophen-2-ylmethyl]amino}cyclobut-3-ene-1,2-dione
Compound 6: 1-(2-chloro-3-fluorophenyl)-3-(4-chloro-2-hydroxy-3-methanesulfox-iminophenyl)urea
Compound 7: 1-(2-chloro-3-fluorophenyl)-3-{4-chloro-2-hydroxy-3-methane[(N-methyl)sulfoximino]phenyl}urea
Compound 8: 3-{2-hydroxy-3-methane[(N-methyl)sulfoximino]phenylamino}-4-[(R)-1-(5-methylfuran-2-yl)propylamino]cyclobut-3-ene-1,2-dione
Compound 9: 3-[4-chloro-3-[N-(2-dimethylaminoethyl)-S-methylsulfonimidoyl]-2-hydroxyanilino]-4-ethoxycyclobut-3-ene-1,2-dione
Compound 10: 3-[4-chloro-3-[N-(2-dimethylaminoethyl)-S-methylsulfonimidoyl]-2-hydroxyanilino]-4-(1-ethylpropylamino)cyclobut-3-ene-1,2-dione
Compound 11: 3-[4-chloro-2-hydroxy-3-[N-(2-methoxyethyl)-S-methylsulfon-imidoyl]anilino]-4-[[(1R)-1-(5-methyl-2-furyl)propyl]amino]cyclobut-3-ene-1,2-dione
Compound 12: (-)-3-{4-chloro-2-hydroxy-3-methane[(N-methyl)sulfoximino]-phenylamino}-4-[(R)-1-(pyridin-2-yl)propylamino]cyclobut-3-ene-1,2-dione
Compound 13: (+)-3-{4-chloro-2-hydroxy-3-methane[(N-methyl)sulfoximino]-phenylaminol}4-[(R)-1-(pyridin-2-yl)propylamino]cyclobut-3-ene-1,2-dione
Compound 14: (-)-3-{2-hydroxy-3-methane[(N-methyl)sulfoximino]phenylamino}-4-[(R)-1-(pyridin-2-yl)propylamino]cyclobut-3-ene-1,2-dione
Compound 15: (-)-3-{2-hydroxy-3-methane[(N-methyl)sulfoximino]phenylamino}-4-[(R)-1-(pyridin-2-yl)propylamino]cyclobut-3-ene-1,2-dione
Compound 16: (+)-3-{2-hydroxy-3-methane[(N-methyl)sulfoximino]phenylamino}-4-[(R)-1-(5-methylfuran-2-yl)propylamino]cyclobut-3-ene-1,2-dione
Compound 17: (+)-3-{2-hydroxy-3-methane[(N-methyl)sulfoximino]phenylamino}-4-[(R)-1-(5-methylfuran-2-yl)propylamino]cyclobut-3-ene-1,2-dione
Compound 18: (-)-3-(2-hydroxy-3-methane[(N-methyl)sulfoximino]phenylamino[4-(1-ethylpropylamino]cyclobut-3-ene-1,2-dione
Compound 19: (+)-3-(2-hydroxy-3-methane[(N-methyl)sulfoximino]phenylamino[4-(1-ethylpropylamino]cyclobut-3-ene-1,2-dione
Compound 20: (+)-3-{4-chloro-2-hydroxy-3-methane[(N-methyl)sulfoximino]-phenylamino}-4-[(R)-1-(5-methylfuran-2-yl)propylamino]cyclobut-3-ene-1,2-dione.

5. A pharmaceutical composition comprising an effective amount of a compound or of a pharmaceutically acceptable salt of said compound according to one of Claims 1 to 4 in combination with a pharmaceutically acceptable support or solvent.

6. The compound according to one of Claims 1-4 or the pharmaceutical composition according to Claim 5 for use as a medicine.

7. The compound according to one of Claims 1-4 or the pharmaceutical composition according to Claim 5 for use in treating α-chemokine-mediated diseases selected from the group comprising neutrophilic dermatoses, especially psoriasis, atopic dermatitis, acne, rosacea, asthma, chronic obstructive pulmonary disease, adult respiratory diseases, arthritis, inflammatory bowel disease, Crohn's disease, graft rejection, mucoviscidosis and skin cancers.

8. The compound or the pharmaceutical composition according to Claim 7 for use in treating dermatological diseases such as neutropilic dermatoses, especially psoriasis, atopic dermatitis, acne and rosacea.
